(19) Europäisches Patentamt
European Patent Office
Office européen des brevets

(11) Veröffentlichungsnummer: **0 001 449**
**B1**

(12) # EUROPÄISCHE PATENTSCHRIFT

(45) Veröffentlichungstag der Patentschrift: **15.04.81**

(21) Anmeldenummer: **78101073.1**

(22) Anmeldetag: **05.10.78**

(51) Int. Cl.[3]: **C 07 C 103/52, A 61 K 37/02**

(54) **Somatostatinanaloge und diese enthaltende pharmazeutische Präparate sowie deren Herstellung.**

(30) Priorität: **11.10.77 US 840922**

(43) Veröffentlichungstag der Anmeldung: **18.04.79 Patentblatt 79/8**

(45) Bekanntmachung des Hinweises auf die Patenterteilung: **15.04.81 Patentblatt 81/15**

(84) Benannte Vertragsstaaten: **BE CH DE FR GB LU NL SE**

(56) Entgegenhaltungen:
**JOURNAL OF THE AMERICAN CHEMICAL SOCIETY, vol. 93, no. 21, 20 October 1971 Easton, Pa., U.S.A.
G. L. ROWLEY et al "On the Specificity of Creatine Kinase. New Glycocyamines and Glycocyamine Analogs Related to Creatine"
Seiten 5542 bis 5551
JOURNAL OF THE CHEMICAL SOCIETY (C), 1971, Bungay, Suffolk, Great Britain
E. ATHERTON et al "Synthesis of Peptides Containing N-2-Amino-ethylglycine—'Reduction Analogues' ",
Seiten 3393 bis 3396**

(73) Patentinhaber: **F. HOFFMANN-LA ROCHE & CO. Aktiengesellschaft
CH-4002 Basel (CH)**

(72) Erfinder: **Dairman, Wallace Mark
Borchester Drive 19
Monsey, N.Y. (US)**
Erfinder: **Felix, Arthur Martin
Central Avenue 257
West Caldwell, N.J. (US)**
Erfinder: **Gallo-Torres, Hugo E.
E. Mt. Pleasant Avenue 215
Livingston, N.J. (US)**
Erfinder: **Heimer, Edgar Philip
Edison Terrace 87
Sparta, N.J. (US)**
Erfinder: **Meienhofer, Johannes Arnold
Glenwood Road 35
Upper Montclair, N.J. (US)**

(74) Vertreter: **Mezger, Wolfgang, Dr. et al,
Grenzacherstrasse 124 Postfach 601
CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 449 B1

# Somatostatinanaloge und diese enthaltende pharmazeutische Präparate sowie deren Herstellung

Die vorliegende Erfindung betrifft neue Analoge des Somatostatins, die einen oder mehrere Aminoäthylglycinreste [N - (2 - Aminoäthyl) - glycin, Aeg] pro Molekül enthalten sowie Verfahren zu deren Herstellung.

Die neuen Verbindungen der vorliegenden Erfindung sind Polypeptide der Formel

X-Lys-Asn-Phe-Phe-A-Lys-Thr-Phe-Thr-Ser-Y I

worin

A L- oder D-Trp,

X H-$(Aeg)_m$-Cys- oder H-$(Aeg)_m$-Ala-Gly-Cys-,

Y -Cys-$(Aeg)_n$-OH oder

X und Y zusammen eine 2-Aminoäthylglycylgruppe in Ringposition und

m und n 0, 1, 2, 3 oder 4, bedeuten, unter der Voraussetzung, dass m + n mindestens 1 ist, deren cyclische Disulfidderivate, Protamin-Zink- und Protamin-Aluminium-Komplexe und physiologisch verträgliche Säureadditionssalze.

Beispiele von erfindungsgemässen Verbindungen sind:

H-Aeg-Cys-Lys-Asn-Phe-Phe-Trp-Lys-Thr-Phe-Thr-Ser-Cys-OH

H-Aeg-Cys-Lys-Asn-Phe-Phe-Trp-Lys-Thr-Phe-Thr-Ser-Cys-OH,

H-Aeg-Aeg-Cys-Lys-Asn-Phe-Phe-Trp-Lys-Thr-Phe-Thr-Ser-Cys-OH,

H-Aeg-Ala-Gly-Cys-Lys-Asn-Phe-Phe-Trp-Lys-Thr-Phe-Thr-Ser-Cys-OH,

Aeg-Lys-Asn-Phe-Phe-Trp-Lys-Thr-Phe-Thr-Ser ,

H-Aeg-Cys-Lys-Asn-Phe-Phe-D-Trp-Lys-Thr-Phe-Thr-Ser-Cys-OH,

H-Aeg-Cys-Lys-Asn-Phe-Phe-D-Trp-Lys-Thr-Phe-Thr-Ser-Cys-OH,

H-Aeg-Aeg-Cys-Lys-Asn-Phe-Phe-D-Trp-Lys-Thr-Phe-Thr-Ser-Cys-OH,

H-Aeg-Aeg-Cys-Lys-Asn-Phe-Phe-D-Trp-Lys-Thr-Phe-Thr-Ser-Cys-OH,

H-Cys-Lys-Asn-Phe-Phe-Trp-Lys-Thr-Phe-Thr-Ser-Cys-Aeg-OH,

H-Cys-Lys-Asn-Phe-Phe-D-Trp-Lys-Thr-Phe-Thr-Ser-Cys-Aeg-OH,

H-Aeg-Cys-Lys-Asn-Phe-Phe-Trp-Lys-Thr-Phe-Thr-Ser-Cys-Aeg-OH,

H-Aeg-Cys-Lys-Asn-Phe-Phe-D-Trp-Lys-Thr-Phe-Thr-Ser-Cys-Aeg-OH.

Die Struktur von Somatostatin wurde von Brazeau et al., Science *179*, 77 (1973), bestimmt. Es sind mehrere Methoden zur Herstellung von Somatostatin in der Literatur beschrieben worden (J.A.C.S. *96*, 2986 (1974); Biochem. Biophys. Res. Comm. *54*, 234 (1973); Helv. Chim. Acta. *57*. 730 (1974); U.S.P. 3,862,925). Inzwischen sind auch eine Anzahl von Somatostatin-Derivaten und Analoga in der Literatur beschrieben worden, beispielsweise in den U.S. Patentschriften Nos. 3,842,066; 3,882,098; 3,904,594; 3,917,581; 3,933,784; 3,988,308; 3,997,517; 3,998,795 und 4,000,259; sowie in Biochem. Biophys. Res. Comm. *65*, 746 (1975) und *73*, 336 (1976) und J. Amer. Chem. Soc. *98*, 2367 (1976).

Aus U.S.P. 4,011,182 (bzw. DOS 26 11 779) und aus U.S.P. 4,133,805 (bzw. DOS 26 56 849) sind cyclische Undecapeptidanaloge des Somatostatins bekannt, die formal dadurch entstehen, dass eine 10 Aminosäurereste umfassende Sequenz des Somatostatins durch eine Aminoalkancarbonsäure, z.B. Glutaminsäure, Aminoadipinsäure, Aminopimelinsäure oder Aminosuberinsäure, zu einem cyclischen Peptid kurzgeschlossen wird. Diese Verbindungen hemmen die Freisetzung von Hypophysen-Wachstumshormonen, z.B. von Somatotropin. Diesen strukturell ähnlichen Verbindungen gegenüber zeichnen sich die vorliegenden neuen Verbindungen durch ein anderes Wirkungsspektrum, nämlich durch Hemmung der Magensäuresekretion und Antiulcerogenität, aus.

Bloom et al. [Lancet, II, 1106 (1974)] haben gezeigt, dass Somatostatin die basale Magensekretion und die Gastrin-Ausschüttung hemmt. Es konnte gezeigt werden, dass die subkutane Verabreichung von Somatostatin an Ratten einen prophylaktischen Effekt auf die Bildung bestimmter Geschwüre hat [Zierden et al., Res. Exp. Med. *168*, 199 (1976)]. Rasche et al. haben berichtet [Klin. Wschr. *54*, 977 (1976)], dass die Infusion von Somatostatin durch Geschwüre des Verdauungstraktes verursachte Blutungen stoppt. Andererseits konnte gezeigt werden, dass die Hemmung der Magensekretion beim Hund durch Somatostatin nur von kurzer Dauer ist [Torchiana et al., Proc. Soc. Exp. Biol. Med. *154*, 449 (1977)]. Ferner ist die Tatsache interessant, dass, während das (D-$Trp^8$)-Somatostatin-Analoge höhere Aktivität als Somatostatin bein der Hemmung des Wachstumshormons, Insulins und Glucagons zeigt, dieses Analoge weniger wirksam bei der Hemmung der durch Pentagastrin induzierten Magensäure-Sekretion ist [Brown et al., Science *196*, 1468 (1977)]. Um klinischtherapeutisch verwertbare Ergebnisse zu erzielen, war es notwendig, nach neuen Analogen des Somatostatins zu suchen, die stärker wirksam sind und/oder längere und spezifischere Wirkung besitzen. Die Verbindungen der vorliegenden Erfindung sind ein Schritt in dieser Richtung.

Die erfindungsgemässen Verbindungen können in an sich bekannter Weise durch Flüssigphasen- oder Festphasen- Synthese hergestellt werden. Gewünschtenfalls können die erhaltenen Verbindungen der Formel I durch milde Oxydation in die entsprechenden cyclischen Disulfide und/oder in physiologisch verträgliche Säureadditionssalze, Protamin-Zink- oder Protamin-Aluminium-Komplexe übergeführt werden.

Im Fall einer konventionellen Flüssigphasen-Peptidsynthese ist die letzte Stufe zur Herstellung einer Verbindung I dadurch gekennzeichnet, dass man die Schutzgruppen aus einem geschützten Polypeptid der Formel

$$X'\text{-Lys}(R^3)\text{-Asn}(R^4)\text{-Phe-A-Lys}(R^3)\text{-Thr}(R^5)\text{-Phe-Thr}(R^5)\text{-Ser}(R^6)\text{-}Y' \qquad II$$

worin

A L- oder D-Trp

X' $R^1$-$(Aeg)_m$-Cys($R^2$)- oder
$R^1$-$(Aeg)_m$(Ala-Gly-Cys($R^2$)-;

Y' -Cys($R^2$)-$(Aeg)_m$-OH oder

X' und Y' zusammen eine gegebenenfalls geschützte 2-Aminoäthylglycylgruppe in Ringposition;

$R^1$ Wasserstoff oder eine konventionelle $\alpha$-Aminoschutzgruppe,

$R^2$ eine konventionelle Schutzgruppe für die Sulfhydrylgruppe des Cysteins;

$R^3$ eine konventionelle Schutzgruppe für die $\varepsilon$-Aminogruppe des Lysins;

$R^4$ Wasserstoff oder eine konventionelle Schutzgruppe für die Carboxamidgruppe des Asparagins;

$R^5$ Wasserstoff oder eine konventionelle Schutzgruppe für die Hydroxylgruppe des Threonins;

$R^6$ Wasserstoff oder eine konventionelle Schutzgruppe für die Hydroxylgruppe des Serins und m und n 0, 1, 2, 3 oder 4 darstellen, unter der Voraussetzung, dass m + n mindestens 1 ist, abspaltet, während man im Falle einer konventionellen Festphasen-Peptidsynthese in letzter Stufe ein an einen festen Träger gebundenes Polypeptid der Formel

$$X''\text{-Lys}(R^3)\text{-Asn}(R^4)\text{-Phe-Phe-A-Lys}(R^3)\text{-Thr}(R^5)\text{-Phe-Thr}(R^5)\text{-Ser}(R^6)\text{-}Y'' \qquad III$$

worin

A L- oder D-Trp;

X'' $R^1$-$(Aeg)_m$-Cys($R^2$)- oder
$R^1$-$(Aeg)_m$-Ala-Gly-Cys($R^2$)-;

Y'' Cys($R^2$)-$(Aeg)_m$- Ⓡ ;

Ⓡ den festen Träger;

$R^1$ Wasserstoff oder eine konventionelle $\alpha$-Aminoschutzgruppe,

$R^2$ eine konventionelle Schutzgruppe für die Sulfhydrylgruppe des Cysteins;

$R^3$ eine konventionelle Schutzgruppe für die $\varepsilon$-Aminogruppe des Lysins;

$R^4$ Wasserstoff oder eine konventionelle Schutzgruppe für die Carboxamidgruppe des Asparagins;

$R^5$ Wasserstoff oder eine konventionelle Schutzgruppe für die Hydroxylgruppe des Threonins;

$R^6$ Wasserstoff oder eine konventionelle Schutzgruppe für die Hydroxylgruppe des Serins und m und n 0, 1, 2, 3 oder 4 darstellen, unter der Voraussetzung, dass m + n mindestens 1 ist, unter

gleichzeitiger Abspaltung vorhandener Schutzgruppen, von Träger abspaltet.

In einer bevorzugten Verfahrensvariante, werden als neue Zwischenverbindungen geschützte Peptide der Formel

$R^1$-Cys($R^2$)-Lys($R^3$)-Asn($R^4$)-Phe-Phe-A-Lys($R^3$)-Thr($R^5$)-Phe-Thr($R^5$)-Ser($R^6$)-Cys($R^2$)-OH IV

verwendet,

worin A wie oben definiert ist;

$R^1$ Wasserstoff oder eine konventionelle $\alpha$-Aminoschutzgruppe darstellt, wie Benzyloxycarbonyl (gegebenfalls am aromatischen Ring substituiert durch 4-Chlor, 2-Brom, 2,4-Dichlor, 4-Nitro, 4-Methoxy, 3,5-Dimethoxy, 4-Methyl, 2,4,6-Trimethyl, 4-Phenylazo, 4-(4-Methoxyphenylazo), 2-(N,N-Dimethylcarbamido) oder 2-Nitro-4,5-dimethoxy), vom Urethantyp, wie 4-Toluolsulfonyläthoxycarbonyl, 9-Fluorenylmethyloxycarbonyl, 5-Benzisoxazolylmethyl-oxycarbonyl, Methylthio, Methylsulfonyläthoxycarbonyl, Isonicotinyloxycarbonyl, Haloäthyloxycarbonyl, Diisopropylmethyloxycarbonyl, Benzhydryloxycarbonyl, Isobornyloxycarbonyl, Dinitrodiphenylmethyloxycarbonyl, t-Butyloxycarbonyl, t-Amyloxycarbonyl, Adamantyloxycarbonyl cyclopentyloxycarbonyl, Methylcyclobutyloxycarbonyl, Methylcyclohexyloxycarbonyl, 2-Arylisopropyloxycarbonyl, wie 2 - (p - Biphenylyl) - isopropyloxycarbonyl, 2 - (4 - Pyridyl) - isopropyloxycarbonyl, eine Acylgruppe, wie Formyl, Trifluoracetyl, Phthaloyl, Benzolsulfonyl, Acetoacetyl, Chloracetyl, 2-Nitrobenzoyl oder 4-Toluolsulfonyl; eine Sulfenylgruppe wie Benzolsulfenyl oder o-Nitrophenylsulfenyl oder eine Aryl-nieder-alkylgruppe, wie Diphenylmethyl und Triphenylmethyl;

$R^2$ eine konventionelle Sulfhydryl-Schutzgruppe wie Benzyl; Methyl-, Methoxy- oder Nitrobenzyl; Trityl; Benzyloxycarbonyl, Benzhydryl, Tetrahydropyranyl, Carboxymethyl, Acetamidomethyl, Benzoyl, Benzylthiomethyl, Aethylcarbamyl, Thioäthyl, p-Methoxybenzyloxycarbonyl, und das Sulfonatsalz;

$R^3$ eine konventionelle Schutzgruppe für die $\varepsilon$-Aminogruppe des Lysins, wie Benzyloxycarbonyl; Halo- oder Nitrobenzyloxycarbonyl, Tosyl, Diisopropylmethoxycarbonyl, t-Amyloxycarbonyl oder t-Butyloxycarbonyl;

$R^4$ Wasserstoff oder eine konventionelle Schutzgruppe für den Carboxamidrest des Asparagins, wie Xanthenyl, 4,4'-Dimethoxyhydryl, 4,4'-Dimethylbenzhydryl, Benzhydryl und t-Butyl;

$R^5$ Wasserstoff oder eine konventionelle Schutzgruppe für die Hydroxylgruppe des Threonins, wie Benzyl, 2,6-Dichlorbenzyl, Benzyloxycarbonyl (gegebenenfalls am aromatischen Ring substituiert durch Halogen, t-Butyl oder Tetrahydropyran-2-yl) und

$R^6$ Wasserstoff oder eine konventionelle Schutzgruppe für die Hydroxylgruppe des Serins, beispielsweise einen der unter $R^5$ definierten Reste, darstellen;

wobei nach dem in Figur 1 dargestellten Schema vorgegangen werden kann.

Die Kopplung der einzelnen Zwischenprodukt-Fragmente bei der Herstellung der Verbindungen der Formel IV kann beispielsweise nach der Methode der N-Hydroxysuccinimidester (Anderson et al., J. Amer. Chem. Soc. *85*, 3039 [1963]), nach der Carbodiimidhydroxybenzotriazol-Methode (König und Geiger, Chem. Ber. *103*, 788 [1970]), nach der Dicyclohexylcarbodiimid-Methode (Sheehan und Hess, J. Amer. Chem. Soc. *77* 1067 [1955]) oder nach der Methode der modifizierten Azidkopplung (Honzl und Rudinger, Coll. Czech. Chem. Comm. *26*, 2333 [1961]) erfolgen.

Die Verbindungen der Formel IV mit $R^1$=H sind besonders wertvoll in der Synthese der neuen $NH_2$-terminalen Aeg-Somatostatin-Analoga der Formel I. Derartige Analoga können zweckmässigerweise durch Kopplung einer Verbindung der Formel IV mit einem geeigneten aktivierten geschützten Aminoäthylglycyl-enthaltenden Rest wie beispielsweise Boc-Aeg(Boc)-OSu, Boc-Aeg(Boc)-Aeg(Boc)OSu oder Boc-Aeg(Boc)-Ala-Gly-OSu und anschliessende Abspaltung der Schutzgruppen, beispielsweise mit Trifluoressigsäure, sowie Behandlung mit wässerigem Quecksilberacetat bei pH 4 hergestellt werden. Die Reinigung der erhaltenen Peptide kann in an sich bekannter Weise erfolgen, z.B. durch Gelfiltration.

Bei der Flüssigphasen-Synthese der erfindungsgemässen Verbindungen sind die folgenden Reste bevorzugt:

$R^1$ = Wasserstoff oder 2-(p-Biphenylyl)-isopropyloxycarbonyl (Bpoc);
$R^2$ = Acetamidomethyl (Acm);
$R^3$ = t-Butyloxycarbonyl (Boc);
$R^4$ = Wasserstoff;
$R^5$ und $R^6$ = t-Butyl.

Die Methoden der Festphasen-Synthese zur Herstellung der erfindungsgemässen Verbindungen der Formel I sind allgemein bekannt und z.B. beschrieben von Merrifield, J. Amer. Chem. Soc. *85*, 2149 (1963). Als feste Träger kommen alle hierfür üblicherweise geeigneten Harze in Frage, vorzugsweise Polystyrol, das mit 0,5 bis 3 Gew.% Divinylbenzol quervernetzt ist und das entweder CHlormethyl- oder Hydroxymethylgruppen enthält. Ein geeignetes Hydroxymethylharz wurde beispielsweise von Bodanszky et al., Chem. Ind. (London) *38*, 1597—98 (1966), beschrieben. Die Herstellung eines chlormethylierten Harzes wurde von Stewart und Young in "Solid Phase Peptide Synthesis", Freeman & Co., San Francisco, 1969, Kapitel 1, Seiten 1—6, beschrieben.

Das für die Festphasen-Synthese der vorliegenden Erfindung verwendete chlormethylierte Polystyrol-Divinylbenzolharz (1% Quervernetzung, 0.037—0,074 mm; 0,90 mMol Cl/g) ist ein Handelsprodukt und wurde in die Hydroxymethylform nach der von Stewart und Young (siehe oben, Seite 27—28) beschriebenen Weise übergeführt.

Jeder Zyklus der Festphasen-Synthese nach der Methode der symmetrischen Anhydride umfasste die Behandlung des Harzes mit den folgenden Reagenzien in der folgenden Reihenfolge:

a) Methylenchlorid, dreimal 5 Minuten,
b) 25% Trifluoressigsäure*-Methylenchlorid, 2 Minuten;
c) 25% Trifluoressigsäure*-Methylenchlorid, 30 Minuten;
d) Methylenchlorid, dreimal je 5 Minuten;
e) 5% Diisopropyläthylamin-Methylenchlorid, 2 Minuten;
f) 5% Diisopropyläthylamin-Methylenchlorid, 10 Minuten;
g) Methylenchlorid, dreimal je 5 Minuten;
h) DMF, dreimal je 5 Minuten;
i) Methylenchlorid, dreimal je 5 Minuten;
j) Mischen der Boc-Aminosäure (6 Aequivalente) mit DCC (3 Aequivalente) während 15 Minuten bei 0°C und 15 Minuten bei 25°C, Filtration und Kopplung während 15 Minuten;
k) 0,4 M Diisopropyläthylamin in Methylenchlorid (3 Aequivalente) 15 Minuten;
l) Methylenchlorid, dreimal je 5 Minuten;
m) DMF, dreimal je 5 Minuten;
n) absolutes Aethanol, dreimal je 5 Minuten;
o) Methylenchlorid, dreimal je 5 Minuten;
p) Diisopropyläthylamin (1 Aequivalent) in Methylenchlorid, 2 Minuten
q) Fluorescamin (1 Aequivalent) in Methylenchlorid, 15 Minuten
r) Methylenchlorid, dreimal je 5 Minuten;
s) absolutes Aethanol, dreimal je 5 Minuten;
t) Methylenchlorid, dreimal je 5 Minuten.

Das erhaltene Peptid kann vom Trägerharz durch einstündige Umsetzung mit Fluorwasserstoff bei 0°C in Gegenwart von Anisol abgespalten werden. Die Reinigung des Rohproduktes kann in der gleichen Weise erfolgen wie bei der Flüssigphasen-Synthese, d.h. durch Gelfiltration. In der Festphasen-Synthese der vorliegenden Erfindung sind für die Seitenketten der Aminosäuren die folgenden Schutzgruppen bevorzugt: p-Methoxybenzyl [PMB] für Cystein, Benzyl für Serin und Threonin, 2-Chlorbenzyloxycarbonyl für Lysin und t-Butyloxycarbonyl für Aminoäthylglycin.

Nach den vorstehend erwähnten Flüssigphasen- bzw. Festphasen-Synthesen werden lineare Verbindungen der Formel I erhalten. Die Ueberführung in eine cyclische Disulfidform kann durch milde Oxydation, vorzugsweise mit Kaliumferricyanid in Gegenwart von Luftsauerstoff, erfolgen.

Die Herstellung von Verbindungen der Formel I mit einem Aminoäthylglycinrest in Ringposition kann zweckmässigerweise nach dem Flüssigphasen-Schema, das in Figur 2 dargestellt ist, erfolgen. Durch Ersatz von L-Trp durch D-Trp an geeigneter Stelle der Peptid-Synthese können die entsprechenden D-Trp-Somatostatin-Analoga hergestellt werden.

Die erfindungsgemässen Verbindungen besitzen wertvolle pharmakologische Eigenschaften. Beispielsweise hemmen sie die Magensekretion, was an nicht-anästhesierten Ratten mit einer akuten Magenfistel gezeigt werden konnte. Sie sind Somatostatin und bekannten Somatostatin-Analoga insofern überlegen, als sie stärker oder länger wirksam sind, spezifische Aktivität besitzen und oral verabreicht werden können.

Es wurde festgestellt, dass bezüglich der Hemmung der Magensekretion z.B. die folgenden Verbindungen dem Somatostatin oder bekannten Analoga überlegen sind:

A H-Aeg-Cys-Lys-Asn-Phe-Phe-Trp-Lys-Thr-Phe-Thr-Ser-Cys-OH

B H-Aeg-Cys-Lys-Asn-Phe-Phe-Trp-Lys-Thr-Phe-Thr-Ser-Cys-OH (Cys–Cys verbrückt)

C H-Aeg-Aeg-Cys-Lys-Asn-Phe-Phe-Trp-Lys-Thr-Phe-Thr-Ser-Cys-OH

D H-Aeg-Cys-Lys-Asn-Phe-Phe-D-Trp-Lys-Thr-Phe-Thr-Ser-Cys-OH

---

*1% 1,2-Aethandithiol wurde der TFA-Lösung zur Abspaltung der Schutzgruppe des Bpoc-Trp-Restes zugesetzt.

E H-Aeg-Cys-Lys-Asn-Phe-Phe-D-Trp-Lys-Thr-Phe-Thr-Ser-Cys-OH

Die Verbindungen D und E zeigten die höchste Aktivität.

Somatostatin-Analoga mit starker und langanhaltender anti-sekretorischer Aktivität, wie z.B. Verbindung D, waren auch, verglichen mit Somatostatin, stärkere Inhibitoren der Bildung bestimmter Geschwüre.

TABELLE 1

Anti-ulcerogene Aktivität von Somatostatin und Verbindung D im RESTRAINT-IMMERSIONS-TEST an der Maus

| | $ED_{50}$ (mg/kg) |
|---|---|
| Somatostatin | 6,70 |
| Verbindung D | 0,98 |

Die erfindungsgemässen Verbindungen können mit verschiedenen nicht-toxischen, inerten, therapeutisch verträglichen festen oder flüssigen Trägern zu Präparaten vereinigt werden, die geeignet sind zur Behandlung von Magen- und/oder Duodenal-Geschwüren. Die Präparate können in an sich bekannter Weise hergestellt werden.

Die Dosierung der aktiven Komponenten hängt von verschiedenen Faktoren ab, wie z.B. der Art und Stärke der zu behandelnden Dysfunktionen. Typische Tagesdosen bei parenteraler Anwendung als Mittel gegen Geschwüre liegen zwischen 0,1 und 100 mg/kg.

Die Verbindungen der Formel I bilden physiologisch verträgliche Säureadditionssalze mit anorganischen und organischen Säuren wie Schwefelsäure, Phosphorsäure, Chlorwasserstoff, Bromwasserstoff, Jodwasserstoff, Salpetersäure, Sulfaminsäure, Zitronensäure, Milchsäure, Benztraubensäure, Oxalsäure, Maleinsäure, Bernsteinsäure, Weinsäure, Zimtsäure, Essigsäure, Trifluoressigsäure, Benzoesäure, Salicylsäure, Gluconsäure und Ascorbinsäure.

Die für die Aminosäuren verwendeten Abkürzungen entsprechen den Nomenklaturregeln, die durch die IUPAC-IUB Commission on Biochemical Nomenclature in Biochem. J. *126*, 773 (1972) publiziert wurden. Alle Aminosäure besitzen L-Konfiguration, sofern nichts anderes angegeben ist.

Die folgenden Beispiele erläutern die Erfindung. Die $R_f$-Werte wurden auf Silicagel-Platten in den folgenden Losungsmittelsystemen (v/v) bestimmt:

(A) Chloroform/Methanol/Essigsäure (85:10:5)
(B) Chloroform/Methanol/Essigsäure (80:2:0,4)
(C) n-Butanol/Essigsäure/Pyridin/Wasser (15:3:10:12)
(D) n-Butanol/0,2 M Essigsäure/Methanol/Pyridin (Oberphase; 4:7:1:1)
(E) n-Propanol/Pyridin/Wasser/Essigsäure/Aethylacetat (5:4:6:1:4)
(F) Chloroform/Methanol/Essigsäure (70:20:5)
(G) Chloroform/Methanol/Essigsäure (85:15:5).

Beispiel 1

a) Eine Lösung von 101,61 g N-Benzyloxycarbonyl-O-butyl-L-threonin-N-hydroxysuccinimidester und 53 g O-t-Butyl-L-serinmethylester-hydrochlorid in 500 ml DMF wurde auf 0°C gekühlt und mit 35 ml Triäthylamin behandelt. Es wurde 1 Stunde bei 0°C und 16 Stunden bei 25°C gerührt, dann zur Trockene eingeengt, in 300 ml Aethylacetat aufgenommen, mit 10%iger $NaHCO_3$-Lösung (3 x 200 ml), gesättigter Natriumchloridlösung (3 x 200 ml), 1 M Zitronensäure (3 x 200 ml) und gesättigter Natriumchloridlösung (3 x 200 ml) extrahiert, über $MgSO_4$ getrocknet, filtriert und zu N - Benzyloxy - carbonyl - O - t - butyl - L - threonin - O - t - butyl - L - serin - methylester in Form eines klaren farblosen Oeles eingeengt. Ausbeute: 112,4 g (96,5%); $[\alpha]_D^{25}$ + 30,34° (c = 1, MeOH); $R_f$ 0,90 (A); 0,94 (B); 0,76 (D).

b) Eine Lösung von 90,8 g des geschützten Dipeptids gemäss Absatz a) in 600 ml Methanol mit einem Gehalt an 5% Pd auf $BaSO_4$ (25 g) und 2 ml Eisessig wurde unter Normaldruck in einem vorgereinigten Wasserstoffstrom (über konz. $H_2SO_4$ getrocknet) während 3 Stunden hydriert. Das Reaktionsgemisch wurde durch Diatomeenerde filtriert, zur Trockene eingeengt und noch dreimal jeweils mit

Benzol aufgenommen und eingeengt. Das erhaltene Oel wurde in 750 ml DMF aufgenommen, auf 0°C gekühlt und mit 77,0 g N - Benzyloxycarbonyl - L - phenylalanin - N - hydroxysuccinimidester umgestezt. Aufarbeitung nach der in Absatz a) beschriebenen Methode lieferte 119 g (96,8%) weissen amorphen N - Benzyloxycarbonyl - L - phenylalanyl - O - t - butyl - L - threonyl - O - t - butyl - L - serin - methylester; $[\alpha]_D^{25}$ + 11,22° (c = 1, DMF); $R_f$ 0,70 (B).

c) Eine Lösung von 11,2 g des geschützten Tripeptids gemäss Absatz b) in 250 ml Methanol mit einem Gehalt an 5% Pd auf $BaSO_4$ (6,75 g) und 1 ml Eisessig wurde wie in Absatz b) beschrieben hydriert und zur Trockene eingeengt. Das erhaltene Oel wurde in 70 ml DMF aufgenommen, auf 0°C abgekühlt und mit 7,32 g N - Benzyloxycarbonyl - O - t - butyl - L - threonin - N - hydroxysuccinimidester gekoppelt. Aufarbeitung wie in Absatz a) beschrieben, anschliessende Chromatographie an einem Hydroxypropylgruppen tragenden Dextrangel (Sephadex® LH 20) unter Verwendung von 95%igem Methanol als Elutionsmittel und Kristallisation aus Aether-Hexan lieferten 12,9 g (91,8%) wiessen amorphen N - Benzyloxycarbonyl - O - t - butyl - L - threonyl - L - phenylalanyl - O - t - butyl - L - threonyl - O - t - butyl - L - serin - methylester, F 113,5—115°C; $[\alpha]_D^{25}$ + 23,63° (c = 1, DMF); $R_f$ 0,75 (B).

d) 123,6 g des gemäss Absatz c) erhaltenen geschützten Tetrapeptids in 650 ml Methanol mit einem Gehalt an 5% Pd auf $BaSO_4$ (25 g) und 2 ml Eisessig wurden wie in Absatz b) beschrieben hydriert und zur Trockene eingeengt. Das erhaltene Oel wurde in 375 ml DMF aufgenommen, auf 0°C gekühlt und mit 76,4 g $N^\alpha$ - Benzyloxycarbonyl - $N^\epsilon$ - t - butyloxycarbonyl - L - lysin - N - hydroxysuccinimidester gekoppelt. Aufarbeitung gemäss Absatz a), Hochdruck-Flüssigkeits-Chromatographie (HPLC) an Kieselgel (8,25 x 80 cm-Säule) mit einem Aethanol/Chloroform-Gradienten und Kristallisation aus Aether-Petroläther lieferten 152,6 g (95,4%) weissen amorphen $N^\alpha$ - Benzyloxycarbonyl - $N^\epsilon$ - t - butyloxycarbonyl - L - lysyl - O - t - butyl - L - threonyl - L - phenylalanyl - O - t - butyl - L - threonyl - O - t - butyl - L - serin - methylester, F. 87—88°C; $[\alpha]_D^{25}$ + 18,19° (c = 1, DMF); $R_f$ 0,54 (B).

e) 133,8 g des gemäss Absatz d) erhaltenen geschützten Pentapeptids in 650 ml Methanol mit einem Gehalt an 5% Pd auf $BaSO_4$ (25 g) und 2 ml Eisessig wurden wie in Absatz d) beschrieben hydriert und zur Trockene eingeengt. Das erhaltene Oel wurde in 600 ml DMF aufgenommen, auf 0°C gekühlt und mit 59,3 g N - [2 - (p - Biphenylyl) - 2 - propyloxycarbonyl] - L - tryptophan umgesetzt, mit nachfolgender Zugabe von 24,66 g Hydroxybenzotriazolhydrat und 27,65 g Dicyclohexylcarbodiimid. Es wurde 1 Stunde bei 0°C und 16 Stunden bei 25°C gerührt, filtriert und das Filtrat zur Trockene eingeengt und durch HPLC an Kieselgel (8,25 x 80 cm-Säule) mit 1-Chlorbutan gereinigt. Kristallisation aus Aethylacetat-Petroläther ergab 146,4 g (84,7%) weissen kristallinen N - [2 - (p - Biphenylyl) - 2 - propyloxycarbonyl] - L - tryptophyl - $N^\epsilon$ - t - butyloxycarbonyl - L - lysyl - O - t - butyl - L - threonyl - L - phenylalanyl - O - t - butyl - L - threonyl - O - t - butyl - L - serin - methylester, F. 111—115°C; $[\alpha]_D^{25}$ + 5,21° (c = 1, DMF); $R_f$ 0,93 (A).

f) 8,19 g des gemäss Absatz e) erhaltenen geschützten Hexapeptidmethylesters in 120 ml n-Butanol/DMF (1:1, v/v) wurden mit 30,7 ml Hydrazinhydrat behandelt und 16 Stunden bei 25°C gerührt. Das Gemisch wurde zur Trockene eingeengt und lieferte nach Kristallisation aus Methanol/-Wasser 7,22 g (88,2%) N- [2 - (p - Biphenylyl) - 2 - propyloxycarbonyl] - L - tryptophyl - $N^\epsilon$ - t - butyloxycarbonyl - L - lysyl - O - t - butyl - L - threonyl - L - phenylalanyl - O - t - butyl - L - threonyl - O - t - butyl - L - serin - hydrazid in Form weisser Körner, F. 176—178°C; $[\alpha]_D^{25}$ + 20,32° (c = 1, $CHCl_3$); $R_f$ 0,72 (A).

g) 7,223 g des gemäss Absatz f) erhaltenen geschützten Hexapeptidhydrazids in 29 ml DMF wurden auf —20°C gekühlt und mit 10,98 ml 3,06 M HCl in THF sowie 1,13 ml Isoamylnitrit behandelt. Das Gemisch wurde 30 Minuten bei —20°C gerührt, auf —25°C abgekühlt und mit 4,7 ml Triäthylamin versetzt. Nach Anstieg der Temperatur auf —20°C wurden 2,561 g S - Acetamidomethyl - L - cystein - hydrochlorid und 3,14 ml Triäthylamin zugesetzt. Es wurde ein pH von 8,0 durch tropfenweise Zugabe von Triäthylamin aufrechterhalten und 1 Stunde bei —20°C sowie 16 Stunden bei 2°C und 5 1/2 Stunden bei 25°C gerührt. Das Gemisch wurde dann zur Trockene eingeengt, der Rückstand mit Wasser behandelt und durch HPLC an Kieselgel mit einem Methanol/Chloroform-Gradienten gereinigt. Kristallisation aus Isopropanol-Petroläther lieferte 6,07 g (74,8%) N - [2 - (p - Biphenylyl) - 2 - propyloxycarbonyl] - L - tryptophyl - $N^\epsilon$ - t - butyloxycarbonyl - L - lysyl - O - t - butyl - L - threonyl - L - phenylalanyl - O - t - butyl - L - threonyl - O - t - butyl - L - seryl - S - acetamidomethyl - L - cystein in Form eines weissen amorphen Pulvers, F. 185,5—187,5°C; $[\alpha]_D^{25}$ —8,18° (c = 1, MeOH); $R_f$ 0,59 (A).

h) 5,558 g des gemäss Absatz g) erhaltenen geschützten Heptapeptids wurden in 364 ml 0,05 M HCl in DMF, enthaltend 20,9 ml Anisol und 3,42 ml Mercaptoäthanol, gelöst. Es wurde 1 Stunde bei 25°C gerührt, zur Trockene eingeengt, mit Aether behandelt und aus Aethanol/Wasser kristallisiert. 3,487 g (75,2%) L - Tryptophyl - $N^\epsilon$ - t - butyloxycarbonyl - L - lysyl - O - t - butyl - L - threonyl - L - phenylalanyl - O - t - butyl - L - threonyl - O - t - butyl - L - seryl - S - acetamidomethyl - L - cystein in Form eines weissen amorphen Pulvers wurden erhalten; F. 200—202°C; $[\alpha]_D^{25}$ +14,63° (c = 0,5, MeOH); $R_f$ 0,72 (C).

i) Eine Lösung von 34,5 g N - t - Butyloxycarbonyl - L - phenylalanin in 400 ml $CH_2Cl_2$ wurde auf 0°C gekühlt und mit 28,0 g L - Phenylalanin - methylester - hydrochlorid sowie 29,5 g Dicyclohexylcarbodiimid und 13,2 g Triäthylamin versetzt. Das Gemisch wurde 2 Stunden bei 0°C und 16 Stunden bei 25°C gerührt, filtriert, zur Trockene eingeengt, mit Aethylacetat aufgenommen und mit Wasser extrahiert. Es wurde über Natriumsulfat getrocknet, filtriert, eingeengt und aus Aethylacetat/Petroläther kristallisiert. Umkristallisation aus $CH_2Cl_2$/Petroläther lieferte 71,73 g (64,7%) weissen kristallinen N - t - Butyloxycarbonyl - L - phenylalanyl - L - phenylalanin - methylester, F. 133—135°C; $R_f$0,91 (A).

j) Eine Lösung des gemäss Absatz i) erhaltenen geschützten Dipeptids (59,0 g) in 3,6 N HCl in THF (3 l) wurde 1 1/2 Stunden bei 25°C gehalten und lieferte nach Einengen zur Trockene und Kristallisation aus THF/Aether 49,8 g (99,4%) L - Phenylalanyl - L - phenylalanyl - methylester - hydrochlorid, F. 199—200°C; $[\alpha]_D^{25}$ +63,5° (c = 1, MeOH). Dieses Salz wurde in 450 ml DMF gelöst, die Lösung auf 5°C gekühlt, mit 13,9 g Triäthylamin neutralisiert und mit 49,86 g N - t - Butyloxycarbonyl - L - asparagin - N - hydroxysuccinimidester umgesetzt. Das Reaktionsgemisch wurde 1 Stunde bei 5°C und 19 Stunden bei 25°C gerührt, filtriert, eingeengt, mit Wasser, Methanol und Aether behandelt und aus Methanol kristallisiert. Es wurden 50,6 g (68,2%) N - t - Butyloxycarbonyl - L - asparaginyl - L - phenylalanyl - L - phenylalanin - methylester erhalten, F. 192—194°C; $R_f$ 0,63 (A).

k) Aus dem gemäss Absatz j) erhaltenen geschützten Tripeptid (26,9 g) wurde durch Behandlung mit 3,86 M HCl in THF (1,5 l) in der in Paragraph j) beschriebenen Weise die Schutzgruppe entfernt. Kristallisation aus Methanol/Aether lieferte ein weisses kristallines Produkt, F. 191—193°C; $[\alpha]_D^{25}$ +9.43° (c = 1, MeOH). 21,5 g dieses Salzes wurden in 270 ml DMF gelöst, mit 21,56 g $N^\alpha$ - Benzyloxycarbonyl - $N^\varepsilon$ - t - butyloxycarbonyl - L - lysin - N - hydroxysuccinimidester und 4,55 g Triäthylamin umgesetzt und 1 Stunde bei 0°C sowie 20 Stunden bei 25°C gehalten. Das Gemisch wurde wie in Absatz j) beschrieben aufgearbeitet und aus Methanol kristallisiert. Es wurden 24,2 g (67,0%) $N^\alpha$ - Benzyloxycarbonyl - $N^\varepsilon$ - t - butyloxycarbonyl - L - lysl - L - asparaginyl - L - phenylalanyl - L - phenylalanin - methylester in Form eines weissen kristallinen Produktes erhalten, F. 208—210°C; $[\alpha]_D^{25}$ −28,92° (c=0,6, DMF); $R_f$ 0,65 (A).

l) Eine Lösung von 5,0 g des gemäss Absatz k) erhaltenen geschützten Tetrapeptids in 150 ml DMF, enthaltend 3,1 g 5% Pd auf $BaSO_4$ und 0,5 ml Eisessig, wurde wie in Absatz b) beschrieben hydriert und zur Trockene eingeengt. Das erhaltene Oel wurde in 85 ml DMF aufgenommen, auf 0°C gekühlt und mit 3,287 g N - [2 - (p - Biphenylyl) - 2 - propyloxycarbonyl] - S - acetamidomethyl - L - cystein - N - hydroxysuccinimidester umgesetzt. Das Gemisch wurde 1 Stunde bei 0°C und 16 Stunden bei 25°C gehalten, zur Trockene eingeengt, mit Wasser behandelt, aus Methanol/Aether kristallisiert und lieferte 4,41 g (65,4%) N - [2 - (p - Biphenylyl) - 2 - propylcarbonyl] - S - acetamidomethyl - L - cycteinyl - $N^\alpha$ - butyloxycarbonyl - L - lysyl - L - asparaginyl - L - phenylalanyl - L - phenylalanin - methylester in Form eines weissen kristallinen Produktes, F. 180—180,5°C; $[\alpha]_D^{25}$ −33,34° (c = 1, DMF); $R_f$ 0,70 (A); 0,86 (C); 0,84 (E).

m) Eine Lösung von 3,745 g des gemäss Absatz 1) erhaltenen geschützten Pentapeptidmethylesters in 60 ml n-Butanol/DMF (1:1, v/v) wurde mit 16,7 ml Hydrazinhydrat behandelt und wie in Absatz f) beschrieben aufgearbeitet. Kristallisation aus DMF/Isopropanol lieferte 3,48 g (93,1%) amorphes N - [2 - (p - Biphenylyl) - 2 - propyloxycarbonyl] - S - acetamidomethyl - L - cysteinyl - $N^\varepsilon$ - t - butyloxycarbonyl - L - lysyl - L - asparaginyl - L - phenylalanyl - L - phenylalanin - hydrazid, F. 216,5—218,5°C; $[\alpha]_D^{25}$ −42,30° (c = 1, DMF); $R_f$ 0,42 (A).

n) Eine Lösung von 2,534 g des gemäss Absatz m) erhaltenen geschützten Pentapeptidhydrazids in 15 ml DMF wurde auf −20°C gekühlt und mit 4,85 ml 2,90 M HCl in THF sowie 0,46 ml Isoamylnitrit versetzt. Dann wurde 30 Minuten bei −20°C gerührt, auf −25°C abgekühlt und 1,97 ml Triäthylamin zugesetzt. Bei einer Temperatur von −20°C wurden dann 3,122 g des gemäss Absatz h) erhaltenen Heptapeptids sowie 0,361 ml Triäthylamin zugesetzt. Es wurde ein pH von 8,0 durch tropfenweise Zugabe von Triäthylamin aufrechterhalten und wie in Absatz g) beschrieben aufgearbeitet. Kristallisation aus DMF/Wasser lieferte 4,811 g (90,8%) N - [2 - (p - Biphenylyl) - 2 - propyloxycarbonyl] - S - acetamidomethyl - L - cysteinyl - $N^\varepsilon$ - t - butyloxycarbonyl - L - lysyl - L - asparaginyl - L - phenylalanyl - L - phenylalanyl - L - tryptophyl - $N^\varepsilon$ - t - butyloxycarbonyl - L - lysyl - O - t - butyl - L - threonyl - L - phenylalanyl - O - t - butyl - L - threonyl - O - t - butyl - L - seryl - S - acetamidomethyl - L - cystein in amorpher Form, $[\alpha]_D^{25}$ −14,40° (c = 1, DMF); $R_f$ 0,27 (A).

o) 4,7 g des gemäss Absatz n) erhaltenen geschützten Dodecapeptids wurden in 200 ml 0,05 M HCl in DMF mit einem Gehalt an 11,3 ml Anisol und 1,9 ml Mercaptoäthanol gelöst. Umsetzung und Aufarbeitung erfolgte wie in Absatz a) beschrieben. Kristallisation aus DMF/Wasser lieferte in quantitativer Ausbeute (4,21 g) kristallines S - Acetamidomethyl - L - cysteinyl - $N^\varepsilon$ - t - butyloxycarbonyl - L - lysyl - L - asparaginyl - L - phenylalanyl - L - phenylalanyl - L - tryptophyl - $N^\varepsilon$ - t - butyloxycarbonyl - L - lysyl - O - t - butyl - L - threonyl - L - phenylalanyl - O - t - butyl - L - threonyl - O - t - butyl - L - seryl - S - acetamidomethyl - L - cystein, F. 210°C (Zers.); $[\alpha]_D^{25}$ −3,00° (c = 1, DMF); $R_f$ 0,21 (F).

p) 750 mg des gemäss Absatz o) erhaltenen geschützten Dodecapeptids wurden in 5 ml DMF bei 0°C mit 770 mg N - t - Butyloxycarbonyl - N - (2 - t - butyloxycarbonylaminoäthyl) - glycin - N - hydroxysuccinimidester umgesetzt. Es wurde N - Methylmorpholin (2,04 ml) zugesetzt, das pH auf 8,0 gehalten und 1 Stunde bei 0°C sowie 16 Stunden bei 25°C gerührt. Dann wurde zur Trockene eingeengt, der Rückstand durch HPLC mit einem Methanol/Chloroform-Gradienten gereinigt und aus

DMF-Wasser ausgefällt. Es wurden 231 mg (26,8%) N - t - Butyloxycarbonyl - N - (2 - t - butyloxycarbonylaminoäthyl) - glycyl - S - acetamidomethyl - L - cysteinyl - $N^{\varepsilon}$ - t - butyloxycarbonyl - L - lysyl - L - asparaginyl - L - phenylalanyl - L - phenylalanyl - L - tryptophyl - $N^{\varepsilon}$ - butyloxycarbonyl - L - lysyl - O - t - butyl - L - threonyl - L - phenylanalyl - O - t - butyl - L - threonyl - O - t - butyl - L - seryl - S - acetamidomethyl - L - cystein erhalten, F. 215°C; $[\alpha]_D^{25}$ −29,74° (c = 0,77, DMF); $R_f$ 0,72 (G).

Der N - t - Butyloxycarbonyl - N - (2 - t - butyloxycarbonylaminoäthyl) - glycin - N - hydroxysuccinimidester wurde folgendermassen hergestellt:

Ein Gemisch aus 7,5 g N - (2 - Aminoäthyl) - glycin, 6,8 g Magnesiumoxid und 27,0 ml t-Butyloxycarbonylazid in 63 ml Dioxan und 63 ml Wasser wurde 25 Stunden bei 50°C gerührt, zur Trockene eingeengt, in 100 ml Wasser aufgenommen, filtriert und dreimal mit je 50 ml Aether extrahiert. Die wässerige Schicht wurde mit Zitronensäure auf pH 3,5 angesäuert und dreimal mit je 50 ml Aethylacetat extrahiert. Der Aethylacetatextrakt wurde mit gesättigter NaCl-Lösung gewaschen, über Magnesiumsulfat getrocknet, filtriert und zur Trockene eingeengt. Der Rückstand wurde zweimal aus Aether-Petroläther kristallisiert und lieferte 13,23 g (65,5%) N - t - Butyloxycarbonyl - N - (2 - t - butyloxycarbonylaminoäthyl) - glycin in Form eines weissen kristallinen Produktes; F. 89—93,5°C; $R_f$ 0,88 (n-BuOH/AcOH/EtOAc/$H_2O$; 1:1:1:1, v/v).

Eine Lösung von 14,1 g N - t - Butyloxycarbonyl - N - (2 - t - butyloxycarbonylaminoäthyl)-glycin in 250 ml $CH_2Cl_2$ und 15 ml DMF wurde auf 0°C gekühlt und mit 5,61 g N - Hydroxysuccinimid sowie 10,1 g Dicyclohexylcarbodiimid versetzt. Das Gemisch wurde 1 Stunde bei 0°C und 16 Stunden bei 25°C gehalten, filtriert, mit $NaHCO_3$—, gesättigter NaCl—, 1 M Zitronensäure- und gesättigter NaCl-Lösung extrahiert, über $MgSO_4$ getrocknet, filtriert und zur Trockene eingeengt. Kristallisation aus Aethylacetat/Petroläther lieferte 17,0 g (92,4%) N - t - Butyloxycarbonyl - N - (2 - t - butyloxycarbonylaminoäthyl) - glycin - N - hydroxysuccinimidester in Form eines weissen kristallinen Produktes, F. 132—136°C.

q) 100 mg des gemäss Absatz p) erhaltenen geschützten Peptids wurden mit 10 ml Trifluoressigsäure unter Stickstoffatmosphäre 2 Stunden bei 25°C behandelt. Das Gemisch wurde zur Trockene eingeengt, mehrere Male mit $CH_2Cl_2$ aufgenommen und wieder eingeengt, der Rückstand mit Wasser aufgenommen und mittels 0,1 M $NH_4OH$ auf pH 4,0 eingestellt. Nach Zusatz von 54,8 mg Quecksilberacetat wurde 1 1/2 Stunden bei 25°C gerührt, 15 Minuten mit $H_2S$ durchgespült, filtriert und das Filtrat lyophilisiert. Gelfiltration an einer 2,5 × 93 cm-Säule eines Dextrangels (Sephadex® G-25) unter Verwendung von 2,0 N Essigsäure/0,01 M $\beta$-Mercaptoäthanol als Elutionsmittel lieferte einen symmetrischen Hauptpeak. Die Fraktionen 61—68 (275—306 ml) wurden lyophilisiert und lieferten 29 mg (41,9%)

Aeg-Cys-Lys-Asn-Phe-Phe-Trp-Lys-Thr-Phe-Thr-Ser-Cys

in Form eines weissen amorphen Pulvers; $R_f$ 0,58 (C).

r) 11,0 mg des gemäss Absatz q) erhaltenen Peptids wurden in 0,62 ml 25%iger Essigsäure gelöst und mit Kaliumferricyanid bis zu einer hellgelben Färbung behandelt. Es wurde 10 Minuten bei 25°C stehengelassen, durch Zusatz von Eisessig auf pH 5,0 eingestellt und 15 Minuten mit einem schwach basischen Ionenaustauscherharz verrührt. Es wurde filtriert und das Filtrat nacheinander auf zwei Säulen aufgebracht, von denen die erste einen schwach basischen Ionenaustauscher (Chloridform; 3 ml) und die zweite einen schwach sauren Ionenaustauscher ($H^+$-Form; 3 ml) enthielt. Die kationenaustauscher-Säule (0,8 × 12 cm) wurde mit 50 ml 5%iger Essigsäure gewaschen und das Peptid mit 50%iger Essigsäure eluiert. Die Fraktionen 1—8 (0—18 ml) wurden lyophilisiert und durch Gelfiltration an einer 0,9 × 54 cm Dextrangel-Säule (Sephadex® G-25) gereinigt. Die Fraktionen 10—16 (20—32 ml) lieferten 6,3 mg (57,8%)

Aeg-Cys-Lys-Asn-Phe-Phe-Trp-Lys-Thr-Phe-Thr-Ser-Cys

in Form eines weissen Pulvers; $R_f$ 0,57 (c); 0,67 (E); 0,25 (D). Die völlige Abwesenheit freier Sulfhydrylgruppen wurde mit Ellman's Reagens überprüft (Arch. Biochem. Biophys., *82*, 70 [1959]).

Beispiel 2

a) 200 mg des geschützten Dodecapeptids aus Beispiel 1, Absatz o) wurden mit 305 mg N - t - Butyloxycarbonyl - N - (2 - t - butyloxycarbonylaminoäthyl) - glycyl - N - 2 - (t - butyloxycarbonylaminoäthyl) - glycin - N - hydroxysuccinimidester in 4 ml DMF bei 0°C wie in Absatz p) beschrieben umgesetzt. Die Reinigung erfolgte durch HPLC mit einem Methanol/Chloroform-Gradienten und Ausfällung aus DMF/Wasser. Es wurden 164 mg (65,6%) N - t - Butyloxycarbonyl - N - (2 - t - butyloxycarbonylaminoäthyl) - glycyl - N - (2 - t - butyloxycarbonylaminoäthyl) -

glycyl - S - acetamidoäthyl - L - cysteinyl - $N^{\varepsilon}$ - t - butyloxycarbonyl - L - lysyl - L - asparaginyl - L - phenylalanyl - L - phenylalanyl - L - tryptophyl - $N^{\varepsilon}$ - t - butyloxycarbonyl - L - lysyl - O - t - butyl - L - threonyl - L - phenylalanyl - O - t - butyl - L - threonyl - O - t - butyl - L - seryl - S - acetamidomethyl - L - cystein in Form eines weissen Feststoffes erhalten; $[\alpha]_D^{25}$ −17,64° (c = 1, DMF); $R_f$ 0,49 (G).

Der N - t - Butyloxycarbonyl - N - (2 - t - butyloxycarbonylaminoäthyl) - glycyl - N - 2 - (t - butyloxycarbonylaminoäthylmethyl) - glycin - N - hydroxysuccinimidester wurde folgendermassen erhalten:

Eine Lösung von 165,1 g Benzyl-p-nitrophenylcarbonat in 1,3 l Dioxan wurde tropfenweise unter Rühren zu einer Lösung von 47,58 g N - (2 - Aminoäthyl) - glycin in 1,3 l Wasser und 1,3 l Dioxan gegeben. Durch Zusatz von 2 N NaOH wurde ein pH von 11 eingestellt. Die Umsetzung erfolgte 16 Stunden bei 25°C. Dann wurde das Gemisch zur Trockene eingeengt, in 1,2 l Wasser aufgenommen und filtriert. Das Filtrat wurde zweimal mit 1,3 l Aethylacetat extrahiert, die wässerige Schicht wurde mit 6 N HCl auf pH 5,5 angesäuert und zweimal mit 1,4 l Aether extrahiert. Die wässerige Schicht wurde dann auf pH 1 angesäuert, zur Trockene eingeengt und nach Aufnahme in Isopropanol nochmals eingeengt. Der Rückstand wurde aus Isopropanol kristallisiert und lieferte 50,7 g (44%) N - (2 - Benzyloxycarbonylaminoäthyl) - glycin in Form weisser Kristalle; F. 176—177°C.

Ein Gemisch aus 940 mg N-(2-Benzyloxycarbonylaminoäthyl)-glycin, 700 mg Magnesiumoxid und 1,02 ml t-Butyloxycarbonylazid in 10 ml Dioxan und 10 ml Wasser wurde 24 Stunden bei 50°C gerührt, zur Trockene eingeengt, in 50 ml Wasser aufgenommen, filtriert und dreimal mit je 50 ml Aether extrahiert. Die wässerige Schicht wurde mit Zitronensäure auf pH 3,5 angesäuert, dreimal mit je 50 ml Aethylacetat extrahiert, die Aethylacetatphase mit gesättigter Natriumchlorid-Lösung gewaschen, über Magnesiumsulfat getrocknet, filtriert und zur Trockene eingeengt. Der Rückstand wurde aus Aethylacetat-Petroläther kristallisiert und lieferte 952 mg (72,7%) N - t - Butyloxycarbonyl - N - (2 - benzyloxycarbonylaminoäthyl) - glycin in Form eines weissen kristallinen Produktes, F. 118—119,5°C.

Eine Lösung von 0,90 g N - t - Butyloxycarbonyl - N - (2 - benzyloxycarbonylaminoäthyl) - glycin in 25 ml Methanol, enthaltend 0,7 g 5% Pd auf $BaSO_4$ wurde 2 1/2 Stunden bei 25°C hydriert. Es wurde filtriert, zur Trockene eingeengt und aus Methanol-Aether kristallisiert. Ausbeute: 430 mg (77,1%) N - t - Butyloxycarbonyl - N - (2 - aminoäthyl) - glycin in Form weisser Kristalle, F. 210—212°C.

Eine Lösung von 727 mg Boc-Aeg(Boc)-OSu und 382 mg N - t - Butyloxycarbonyl - N - (2 - aminoäthyl) - glycin in 15 ml DMF wurde mit 0,20 ml N-Methylmorpholin versetzt und 16 Stunden bei 25°C gerührt. Durch Zusatz von N-Methylmorpholin wurde das pH auf 7,5—8 gehalten. Dann wurde zur Trockene eingeengt, in Aethylacetat aufgenommen, zweimal mit 25 ml 0,1 M Zitronensäure und gesättigter NaCl-Lösung extrahiert, über Magnesiumsulfat getrocknet, filtriert, zur Trockene eingeengt und mit Pentan behandelt. Es wurden 873 mg (93,3%) N - t - Butyloxycarbonyl - N - (2 - t - butyloxycarbonylaminoäthyl) - glycyl - N - (2 - t - butyloxycarbonylaminoäthyl) - glycin in Form eines weissen amorphen Pulvers erhalten

Eine Lösung von 778 mg N - t - Butyloxycarbonyl - N - (2 - t - butyloxycarbonylaminoäthyl) - glycyl - N - (2 - t - butyloxycarbonylaminoäthyl) - glycin in 12 ml $CH_2Cl_2$ und 1 ml DMF wurde auf 0°C gekühlt und mit 196 mg N-Hydroxysuccinimid sowie 351 mg Dicyclohexylcarbodiimid versetzt. Es wurde 1 Stunde bei 0°C und 16 Stunden bei 25°C gehalten, filtriert, zur Trockene eingeengt und aus Aethylacetat-Petroläther kristallisiert. Ausbeute: 689 mg (74,6%) N - t - Butyloxycarbonyl - N - (2 - t - butyloxycarbonylaminoäthyl) - glycyl - N - (2 - t - butyloxycarbonylaminoäthyl) - glycin - N - hydroxysuccinimidester in Form weisser Kristalle, F. 90,5—94°C.

b) 60,6 mg des gemäss Absatz a) erhaltenen geschützten Peptids wurden mit 10 ml Trifluoressigsäure umgesetzt, wie in Beispiel 1, Absatz q) beschrieben, und anschliessend mit 30,6 mg Quecksilberacetat behandelt. Die Aufarbeitung erfolgte wie in Beispiel 1 beschrieben und das Produkt wurde durch Gelfiltration an einer 2,5 x 93 cm-Saüle eines Dextrangels (Sephadex® G-25) gereinigt. Die Fraktionen 54—67 (243—302 ml) wurden lyophilisiert und lieferten 15 mg (36,5%)

Aeg-Aeg-Cys-Lys-Asn-Phe-Phe-Trp-Lys-Thr-Phe-Thr-Ser-Cys

in Form eines weissen Pulvers; $R_f$ 0,56 (C); 0,72 (E).

Beispiel 3

a) Eine Lösung von 164 mg des gemäss Beispiel 1, Absatz o) erhaltenen geschützten Dodecapeptids wurde mit 221 mg N - t - Butyloxycarbonyl - N - (2 - t - butyloxycarbonylaminoäthyl) - glycyl - L - alanyl - glycin - N - hydroxysuccinimidester in 2 ml DMF bei 0°C wie in Absatz p) von Beispiel 1 beschrieben umgesetzt. Die Reinigung des Rohproduktes erfolgte durch HPLC mit einem Methanol/Chloroform-Gradienten und Ausfällung aus DMF/Wasser. Es wurden 208 mg (37,0%) N - t - Butyloxycarbonyl - N - (2 - t - butyloxycarbonylaminoäthyl) - glycyl - L - alanyl - glycyl - S - acetamidomethyl - L - cysteinyl - $N^{\varepsilon}$ - t - butyloxycarbonyl - L - lysyl - L - asparaginyl - L -

phenylalanyl - L - phenylalanyl - L - tryptophyl - $N^{\varepsilon}$ - t - butyloxycarbonyl - L - lysyl - O - t - butyl - L - threonyl - L - phenylalanyl - O - t - butyl - L - threonyl - O - t - butyl - L - seryl - S - acetamidomethyl - L - cystein als weisser Feststoff erhalten; $R_f$ 0,39 (G).

Der N - t - Butyloxycarbonyl - N - (2 - t - butyloxycarbonylaminoäthyl) - glycyl - L - alanyl - glycin - N - hydroxysuccinimidester wurde folgendermassen hergestellt:

Eine Lösung von 1,039 g N - t - Butyloxycarbonyl - N - (2 - t - butyloxycarbonylaminoäthyl) - glycin - N - hydroxysuccinimidester und 223 mg L-Alanin in 5 ml DMF wurde mit 0,35 ml N-Methylmorpholin behandelt und 48 Stunden bei 25°C gerührt. Es wurde weiteres N-Methylmorpholin zugesetzt, um ein pH von 7,5—8 aufrechtzuerhalten, zur Trockene eingeengt, in Aethylacetat aufgenommen, zweimal mit 25 ml 0,5 M Zitronensäure und gesättigter NaCl-Lösung extrahiert, über Magnesiumsulfat getrocknet, filtriert, zur Trockene eingeengt und aus Aether-Petroläther kristallisiert. Ausbeute: 567 mg (58,2%) N - t - Butyloxycarbonyl - N - (2 - t - butyloxycarbonylaminoäthyl) - glycyl - L - alanin; F. 106—113°C.

Eine Lösung von 200 mg N - t - Butyloxycarbonyl - N - (2 - t - butyloxycarbonylaminoäthyl) - glycyl - L - alanin in 2 ml THF wurde auf —15°C gekühlt und mit 58 $\mu$l N-Methylmorpholin sowie 68 $\mu$l Chlorameisensäureisobutylester versetzt. Es wurde 1 Minute bei —15°C gerührt, auf —20°C abgekühlt, mit 147 mg TFA·H-Gly-OSu in 0,5 ml THF und 58 $\mu$l N-Methylmorpholin versetzt und 1 Stunde bei —15°C sowei 4 Stunden bei 25°C gerührt. Dann wurde zur Trockene eingeengt, in Aethylacetat aufgenommen, mit 5% $NaHCO_3$—, NaCl— und gesättigter 1 M Zitronensäure-Lösung extrahiert, über Magnesiumsulfat getrocknet, filtriert, zur Trockene eingeengt und unter vermindertem Druck getrocknet. Ausbeute: 208 mg (74,4%) N - t - Butyloxycarbonyl - N - (2 - t - butyloxycarbonylaminoäthyl) - glycyl - L - alanyl - glycin - N - hydroxysuccinimidester in Form eines weissen Feststoffes.

b) 52 mg des gemäss Absatz a) erhaltenen geschützten Peptids wurden mit 10 ml Trifluoressigsäure, wie in Beispiel 1, Absatz q) beschrieben, umgesetzt und mit 25,5 mg Quecksilberacetat behandelt. Aufarbeitung erfolgte wie in Beispiel 1 beschrieben und das Rohprodukt wurde durch Gelfiltration an einer 2,5 x 93 cm-Säule eines Dextrangels (Sephadex® G-25) gereinigt. Die Fraktionen 55—67 (248—302 ml) wurden lyophilisiert und an einer 0,9 x 54 cm-Säule des gleichen Dextrangels rechromatographiert. Die Fraktionen 9—12 (18—24 ml) wurden lyophilisiert und lieferten 14,5 mg (41,7%)

Aeg-Ala-Gly-Cys-Lys-Asn-Phe-Phe-Trp-Lys-Thr-Phe-Thr-Ser-Cys

als weisses amorphes Pulver; $R_f$ 0,57 (C); 0,76 (E); 0,32 (D).

## Beispiel 4

a) 4,019 g des geschützten Tetrapeptid-methylesters von Beispiel 1, Absatz k) in 50 ml n-Butanol/DMF (1:1, v/v) wurden mit 26 ml Hydrazinhydrat umgesetzt. Das Gemisch wurde 22 Stunden bei 25°C gerührt, zur Trockene eingeengt und aus DMF-Isopropanol kristallisiert. Es wurden 3,551 g (88%) $N^{\alpha}$ - Benzyloxycarbonyl - $N^{\varepsilon}$ - t - butyloxycarbonyl - L - lysyl - L - asparaginyl - L - phenylalanyl - L - phenylalanin - hydrazin in amorpher Form erhalten; F. 233—234,5°C; $[\alpha]_D^{25}$ —40,20 (c = 1, DMF); $R_f$ 0,85 (G); 0,82 (C).

b) 1,935 g des gemäss Beispiel 1, Absatz e) erhaltenen geschützten Hexapeptids wurden in 134 ml 0,053 M HCl in DMF, enthaltend 8,19 ml Anisol und 1,25 ml Mercaptoäthanol, gelöst. Es wurde 1 Stunde bei 25°C gerührt, zur Trockene eingeengt und mit Aether und Petroläther behandelt. Ausbeute: 1,514 g (89,8%) L - Tryptophyl - $N^{\varepsilon}$ - t - butyloxycarbonyl - L - lysyl - O - t - butyl - L - threonyl - L - phenylalanyl - O - t - butyl - L - threonyl - O - t - butyl - L - serin - methylester - hydrochlorid als amorpher Feststoff; F. 155—159°C; $[\alpha]_D^{25}$ +15,15° (c = 1, MeOH).

c) Eine Lösung von 1,457 g des gemäss Absatz a) erhaltenen Tetrapeptidhydrazids in 28 ml DMF wurde auf —20°C gekühlt, mit 7,5 ml 1,455 M HCl in THF und 0,747 ml Isoamylnitrit versetzt und 30 Minuten bei —20°C gerührt. Das Gemisch wurde auf —25°C gekühlt, mit 1,51 ml Triäthylamin versetzt, auf —20°C erwärmt und mit 2,242 g des gemäss Absatz b) hergestellten Hexapeptids sowie 0,462 ml Triäthylamin versetzt. Durch tropfenweisen Zusatz von Triäthylamin wurde ein pH von 8,0 eingehalten. Die Aufarbeitung erfolgte wie in Beispiel 1, Absatz f) beschrieben. Kristallisation aus Aethanol lieferte 2,424 g (73,1%) $N^{\alpha}$ - Benzyloxycarbonyl - $N^{\varepsilon}$ - t - butyloxycarbonyl - L - lysyl - L - asparaginyl - L - phenylalanyl - L - phenylalanyl - L - tryptophyl - $N^{\varepsilon}$ - t - butyl - oxycarbonyl - L - lysyl - O - t - butyl - L - threonyl - L - phenylalanyl - O - t - butyl - L - threonyl - O - t - butyl - L - serin - methylester als weissen amorphen Feststoff; F. 223—226°C; $R_f$ 0,81 (C); 0,82 (D).

d) Ein Gemisch aus 2,12 g des gemäss Absatz c) erhaltenen geschützten Decapeptids, 15 ml DMF, 30 ml Methanol und 1,5 g 5% Pd auf $BaSO_4$ wurde unter Normaldruck hydriert und wie in Beispiel 1, Absatz b) beschrieben, aufgearbeitet. Kristallisation aus DMF/Wasser lieferte 1,32 g (67,2%) $N^{\varepsilon}$ - t - Butyloxycarbonyl - L - lysyl - L - asparaginyl - L - phenylalanyl - L - phenylalanyl - L - tryptophyl - $N^{\varepsilon}$ - t - butyloxycarbonyl - L - lysyl - O - t - butyl - L - threonyl - L - phenylalanyl - O - t - butyl - L - threonyl - O - t - butyl - L - serin - methylester in Form eines weissen Feststoffes; F. 210—213,5°C; $[\alpha]_D^{25}$ —9,51° (c = 1,1, DMF); $R_f$ 0,67 (D).

e) Eine Lösung von 476 mg des vorstehend erhaltenen geschützten Decapeptids in 5,6 ml DMF

wurde bei 0°C mit 312 mg N - [2 - (p - Biphenylyl) - 2 - propyloxycarbonyl] - N - (2 - t - butyloxycarbonylaminoäthyl) - glycin - N - hydroxysuccinimidester umgesetzt. Es wurde N-Methylmorpholin (31,7 μl) zugesetzt, das pH auf 8,0 gehalten und das Reaktionsgemisch wie in Beispiel 1, Absatz p) beschrieben, aufgearbeitet. Reinigung durch HPLC mit einem Methanol/Chloroform-Gradienten und anschliessende Fällung aus DMF/Wasser lieferte 233 mg (39,0%) N - [2 - (p - Biphenylyl) - 2 - propyloxycarbonylaminoäthyl] - N - (2 - t - butyloxycarbonylaminoäthyl) - glycyl - $N^{\varepsilon}$ - t - butyloxycarbonyl - L - lysyl - L - asparaginyl - L - phenylalanyl - L - phenylalanyl - $N^{\varepsilon}$ - t - butyloxycarbonyl - L - lysyl - O - t - butyl - L - threonyl - L - phenylalanyl - O - t - butyl - L - threonyl - O - t - butyl - L - serin - methylester als weissen Feststoff; F. 214—217°C; $R_f$ 0,82 (C); 0,77 (D); 0,83 (E).

f) Eine Lösung von 220 mg des gemäss Absatz e) erhaltenen geschützten Peptids in 4 ml n-Butanol/DMF (1:1, v/v) wurde mit 0,499 ml Hydrazinhydrat versetzt und 21 Stunden bei 25°C gerührt. Das Reaktionsgemisch wurde zur Trockene eingeengt und lieferte nach Kristallisation aus DMF-Isopropanol 191 mg (86,8%) N - [2 - (p - Biphenylyl) - 2 - propyloxycarbonylaminoäthyl] - N - (2 - t - butyloxycarbonylaminoäthyl) - glycyl - $N^{\varepsilon}$ - t - butyloxycarbonyl - L - lysyl - L - asparaginyl - L - phenylalanyl - L - phenylalanyl - $N^{\varepsilon}$ - t - butyloxycarbonyl - L - lysyl - O - t - butyl - L - threonyl - L - phenylalanyl - O - t - butyl - L - threonyl - O - t - butyl - L - serin - hydrazid in Form eines amorphen weissen Feststoffs, F. 215—220°C; $R_f$ 0,78 (C).

g) Eine Lösung von 171 mg des oben erhaltenen geschützten Peptidhydrazids in 7,64 ml 0,05 M HCl in DMF wurde 1 Stunde bei 25°C stehengelassen und zur Trockene eingeengt. Der Rückstand wurde in 2 ml DMF gelöst, auf —20°C abgekühlt und mit 0,31 ml 1,77 M HCl in THF sowie 19,4 μl Isoamylnitrit versetzt. Nach 30-minütigem Rühren bei —20°C wurde das Gemisch auf —25°C abgekühlt und mit 174 ml vorgekühltem DMF verdünnt. Nach Zusatz von 97,3 μl Diisopropyläthylamin wurde durch tropfenweisen weiteren Zusatz von Diisopropyläthylamin ein pH von 8,0 eingehalten und 1 Stunde bei —20°C sowei 19 Stunden bei 2°C gerührt. Es wurde zur Trockene eingeengt, mit Wasser behandelt und aus DMF/Wasser ausgefällt. Ausbeute: 93,2 mg (55%) Cyclo - [N - (2 - t - butyloxycarbonylaminoäthyl) - glycyl - $N^{\varepsilon}$ - t - butyloxycarbonyl - L - lysyl - L - asparaginyl - L - phenylalanyl - L - phenylalanyl - L - tryptophyl - $N^{\varepsilon}$ - t - butyloxycarbonyl - L - lysyl - O - t - butyl - L - threonyl - L - phenylalanyl - O - t - butyl - L - threonyl - O - t - butyl - L - seryl] in Form eines weissen amorphen Feststoffes, F. 220—225°C.

h) 40 mg des gemäss Absatz g) erhaltenen geschützten cyclischen Peptids wurden unter Stickstoffatmosphäre bei 25°C 2 Stunden lang mit 10 ml Trifluoressigsäure behandelt. Das Gemisch wurde zur Trockene eingeengt, mehrere Male mit $CH_2Cl_2$ aufgenommen und wieder eingeengt und aus Wasser lyophilisiert. Es wurde durch Gelfiltration an einer 2,5 x 90 cm-Säule eines Dextrangels (Sephadex® G-15) unter Verwendung von 2,0 N Essigsäure/0,01 M β-Mercaptoäthanol als Elutionsmittel gereinigt. Die Fraktionen 42—60 (189—270 ml) wurden lyophilisiert und weiter durch Gelfiltration an einer 1,7 x 74 cm-Säule Dextrangels (Sephadex® G-25) wie oben beschrieben, gereinigt. Die Fraktionen 33—41 (79—98 ml) wurden lyophilisiert und lieferten 9,2 mg (30,7%)

┌Aeg-Lys-Asn-Phe-Phe-Trp-Lys-Thr-Phe-Thr-Ser┐;

$R_f$ 0,67 (C); 0,27 (D); 0,80 (E).

## Beispiel 5

a) N - t - Butyloxycarbonyl - S - p - methoxybenzyl - L - cystein wurde an hydroxymethyliertes Polystyrol-Divinylbenzol-Harz unter Verwendung von Dicyclohexylcarbodiimid gekoppelt. Abspaltung der Schutzgruppe mit 25%iger Trifluoressigsäure (TFA) in $CH_2Cl_2$ lieferte TFA·Cys(PMB)-Harz. 2,98 g dieses Harzes wurden nach der vorne beschriebenen Festphasenmethode nacheinander mit jeweils 4,0 Aequivalenten von Boc-Ser(Bzl)-OH, Boc-Thr(Bzl)-OH, Boc-Phe-OH, Boc-Lys(2-ClZ)-OH, Boc-D-Trp-OH, Boc-Phe-OH, Boc-Phe-OH, Boc-Asn-ONP, Boc-Lys(2-ClZ)-OH, Boc-Cys(PMB)-OH und Boc-Aeg(Boc)-OH gekoppelt. Die Kopplungen gingen jeweils innerhalb von 2 Stunden in Anwesenheit von 4,0 Aequivalenten Dicyclohexylcarbodiimid vor sich (mit Ausnahme von Boc-Asn-ONP, das direkt innerhalb von 24 Stunden koppelte). Das Peptidharz (4,73 g) wurde bei 0°C innerhalb von 45 Minuten mit ca. 50 ml HF, enthaltend 4,26 ml Anisol, gespalten. Der Fluorwasserstoff wurde unter vermindertem Druck entfernt und der Rückstand mit Aether gewaschen, in 0,1 M Essigsäure (enthaltend 2-Mercaptoäthanol) extrahiert und lyophilisiert. Ausbeute 0,773 g. 206 mg dieses Materials wurde durch Gelfiltration an einer 2,5 x 90 cm-Säule eines Dextrangels (Sephadex® G-15) gereinigt. Die Elution erfolgte mit 2,0 M Essigsäure/0,01 M 2-Mercaptoäthanol, wobei die Fraktionen 42—56 (189—252 ml) gesammelt und lyophilisert wurden. Rechromatographie an einer 1,7 x 74 cm-Säule eines Dextrangels (Sephadex® G-25) lieferte einen symmetrischen Hauptpeak. Die Fraktionen 34—42 (82—101 ml) wurden lyophilisiert und lieferten 32,3 mg

Aeg-Cys-Lys-Asn-Phe-Phe-D-Trp-Lys-Thr-Phe-Thr-Ser-Cys

in Form eines weissen amorphen Pulvers; $R_f$ 0,58 (C); 0,72 (E); 0,35 (D).

b) 15,0 mg des gemäss Absatz a) erhaltenen Peptids wurden in 0,93 ml 25%iger Essigsäure gelöst und mit Kaliumferricyanid behandelt. Die Lösung wurde, wie in Beispiel 1, Absatz f) beschrieben, aufgearbeitet und das Rohprodukt durch Gelfiltration an einer 1,7 x 74 cm-Säule eines Dextrangels (Sephadex® G-25) gereinigt. Die Fraktionen 32—45 (70—79 ml) lieferten

Aeg-Cys-Lys-Asn-Phe-Phe-D-Trp-Lys-Thr-Phe-Thr-Ser-Cys

in Form eines weissen Pulvers: $R_f$ 0,55 (C); 0,68 (E); 0,27 (D); Ausbeute: 6,7 mg (44,7%). Die völlige Abwesenheit freier Sulfhydrylgruppen wurde mit Ellman's Reagens überprüft.

## Beispiel 6

a) 2,98 g des TFA·Cys(PMB)-Harzes aus Beispiel 5 (a) wurde nach der Festphasenmethode mit jeweils 4,0 Aequivalenten der folgenden geschützten-Aminosäuren umgesetzt: Boc-Ser(Bzl)-OH, Boc-Thr(Bzl)-OH, Boc-Phe-OH, Boc-Lys(2-ClZ)-OH, Boc-D-Trp-OH, Boc-Phe-OH, Boc-Phe-OH, Boc-Asn-ONP, Boc-Lys(2-ClZ)-OH, Boc-Cys(PMB)-OH, Boc-Aeg(Z)-OH und Boc-Aeg(Boc)-OH. Die Umsetzungen erfolgten in der in Beispiel 5 a) beschriebenen Weise. Das Peptidharz wurde dann innerhalb 1 Stunde mit ca. 45 ml HF, enthaltend 4,2 ml Anisol, bei 0°C gespalten. Der Fluorwasserstoff wurde unter vermindertem Druck entfernt und der Rückstand wie in Beispiel 5 a) beschrieben aufgearbeitet, zu 915 mg Rohprodukt. 250 mg dieses Produktes wurden durch Gelfiltration an einer 2,5 x 90 cm-Säule eines Dextrangels (Sephadex® G-15) gereinigt. Die Fraktionen 45—52 (189—245 ml) wurden lyophilisiert (Ausbeute: 56 mg) und an einer 1,7 x 74 cm-Säule eines Dextrangels (Sephadex® G-25) rechromatographiert. Die Fraktionen 17—22 (76—99 ml) wurden lyophilisiert und lieferten 34 mg gereinigtes Produkt. Die letzte Reinigung erfolgte durch Gelfiltration an einer 1,7 x 74 cm-Säule eines Dextrangels (Sephadex® G-25) (Fraktionen 27—40 [75—100 ml]) und lieferte 29,5 mg

Aeg-Aeg-Cys-Lys-Asn-Phe-Phe-D-Trp-Lys-Thr-Phe-Thr-Ser-Cys

in Form eines weissen amorphen Pulvers; $R_f$ 0,50 (C); 0,21 (D); 0,76 (E).

b) 20 mg des gemäss Absatz a) erhaltenen Peptids wurden in 1,1 ml 25%iger Essigsäure gelöst, mit 34 ml Wasser verdünnt und mit Kaliumferricyanid behandelt. Die Aufarbeitung der Lösung erfolgte in der in Beispiel 1, Absatz r) beschriebenen Weise und das erhaltene Rohprodukt wurde durch Gelfiltration an einer 1,7 x 74 cm-Säule eines Dextrangels (Sephadex® G-25) gereinigt. Die Fraktionen 26—37 (64—91 ml) lieferten

Aeg-Aeg-Cys-Lys-Asn-Phe-Phe-D-Trp-Lys-Thr-Phe-Thr-Ser-Cys

in Form eines weissen Pulvers; $R_f$ 0,51 (C); 0,22 (D); 0,68 (E); Ausbeute: 12,4 mg (62%). Die völlige Abwesenheit freier Sulfhydrylgruppe wurde mit Ellman's Reagens geprüft.

## Beispiel 7

N - Benzoyloxycarbonyl - N - (2 - t - butyloxycarbonylaminoäthyl) - glycin wurde an das hydroxymethylierte Polystyrol-Divinylbenzol-Harz unter Verwendung von Dicyclohexylcarbodiimid also Kondensationsmittel gekoppelt. Entfernung der Schutzgruppe mit 25%iger TFA in $CH_2Cl_2$ lieferte TFA·Aeg(Z)-Harz. 2,83 g dieses Harzes wurden nach der Festphasen-Technik unter Verwendung der Methode der symmetrischen Anydride wie vorne beschrieben nacheinander mit jeweils 5,71 Aequivalenten der folgenden geschützten Anhydride umgesetzt: $[Boc-Cys(PMB)]_2O$, $[Boc-Ser(Bzl)]_2O$, $[Boc-Thr(Bzl)]_2O$, $[Boc-Phe]_2O$, $[Boc-Thr(Bzl)]_2O$, $[Boc-Lys(2-ClZ)]_2O$, $[Boc-Trp]_2O$, $[Boc-Phe]_2O$, $[Boc-Phe]_2O$, Boc-Asn-ONP, $[Boc-Lys(2-ClZ)]_2O$ und $[Boc-Cys(PMB)]_2O$. 4,4 g des erhaltenen Peptidharzes wurden innerhalb von 45 Minuten bei 0°C mit etwa 45 ml Fluorwasserstoff, enthaltend 4,01 ml Anisol, gespalten. Der Fluorwasserstoff wurde unter vermindertem Druck entfernt und die Aufarbeitung erfolgte wie in Beispiel 5 a) beschrieben zu 1,09 g Rohprodukt. 376 mg dieses Produktes wurden durch Gelfiltration an einer 2,5 x 90 cm-Säule eines Dextrangels (Sephadex® G-15) gereinigt. Die Fraktionen 50—56 (225—252 ml) wurden lyophilisiert und an einer 1,7 x 74 cm-Säule eines Dextrangels (Sephadex® G-25) rechromatographiert. Die Fraktionen 37—42 (89—101 ml) wurden wiederum lyophilisiert und lieferten 26 mg gereinigtes Produkt. Die letzte Reinigung erfolgte durch Gelfiltration an einer 1,7 x 74 cm-Säule eines Dextrangels (Sephadex® G-25) [Fraktionen 31—39 (74—94 ml)] und lieferte 17,8 mg

Cys-Lys-Asn-Phe-Phe-Trp-Lys-Thr-Phe-Thr-Ser-Cys-Aeg

in Form eines weissen amorphen Pulvers; $R_f$ 0,59 (C); 0,26 (D); 0,77 (E).

Beispiel 8

Unter Verwendung der in Beispiel 7 beschriebenen Methode und der gleichen Aufarbeitung wurden 0,906 mg rohes

Cys-Lys-Asn-Phe-Phe-D-Trp-Lys-Thr-Phe-Thr-Ser-Cys-Aeg

hergestellt. 338 mg dieses Produktes wurden durch Gelfiltration, wie in Beispiel 7 beschrieben, gereinigt und lieferten 21,8 mg der genannten Verbindung in Form eines weissen amorphen Pulvers; $R_f$ 0,51 (C); 0,26 (D); 0,82 (E).

Beispiel 9

2,83 g des in Beispiel 7 beschriebenen TFA·Aeg(Z)-Harzes wurden nach der Festphasen-Technik unter Verwendung der Methode der symmetrischen Anhydride behandelt und nacheinander mit jeweils 5,71 Aequivalenten der folgenden geschützten Aminosäureanhydride gekoppelt: $[Boc-Cys(PMB)]_2O$, $[Boc-Ser(Bzl)]_2O$, $[Boc-Thr(Bzl)]_2O$, $[Boc-Phe]_2O$, $[Boc-Thr(Bzl)]_2O$, $[Boc-Lys(2-ClZ)]_2O$, $[Boc-Trp]_2O$, $[Boc-Phe]_2O$, $[Boc-Phe]_2O$, Boc-Asn-ONP, $[Boc-Lys(2-ClZ)]_2O$, $[Boc-Cys(PMB)]_2O$ und $[Boc-Aeg(Boc)]_2O$. 4,54 g des Peptidharzes wurden bei 0°C innerhalb von 45 Minuten durch Behandlung mit etwa 45 ml Fluorwasserstoff, enthaltend 4,01 ml Anisol, gespalten. Der Fluorwasserstoff wurde unter vermindertem Druck entfernt und die Aufarbeitung des Rückstandes erfolgte nach der in Beispiel 5 a) angegebenen Methode zu 1,22 g Rohprodukt. 963 mg des Rohproduktes wurde durch Gelfiltration an einer 2,5 x 90 cm-Säule eines Dextrangels (Sephadex® G-15) gereinigt. Die Fraktionen 58—71 (261—320 ml) wurden lyophilisiert und an der gleichen Säule rechromatographiert. Die Fraktionen 44—55 (198—248 ml) wurden lyophilisiert und lieferten 72,1 mg gereinigtes Produkt. Die letzte Reinigung erfolgte durch Gelchromatographie an einer 1,7 x 74 cm-Säule eines Dextrangels (Sephadex® G-25) [Fraktionen 34—42 (82—100 ml)] und lieferte 43,1 mg

Aeg-Cys-Lys-Asn-Phe-Phe-Trp-Lys-Thr-Phe-Thr-Ser-Cys-Aeg

in Form eines weissen amorphen Produktes; $R_f$ 0,58 (C); 0,15 (D); 0,71 (E).

Beispiel 10

In Analogie zu dem in Beispiel 9 beschriebenen Verfahren wurden 1,09 g rohes

Aeg-Cys-Lys-Asn-Phe-Phe-D-Trp-Lys-Thr-Phe-Thr-Ser-Cys-Aeg

hergestellt. 240 mg des Rohproduktes wurden auf die in Beispiel 9 beschriebene Weise gereinigt zu 25,4 mg der genanten Verbindung in Form eines weissen amorphen Produktes; $R_f$ 0,56 (C); 0,21 (D); 0,72 (E).

FIGUR 1

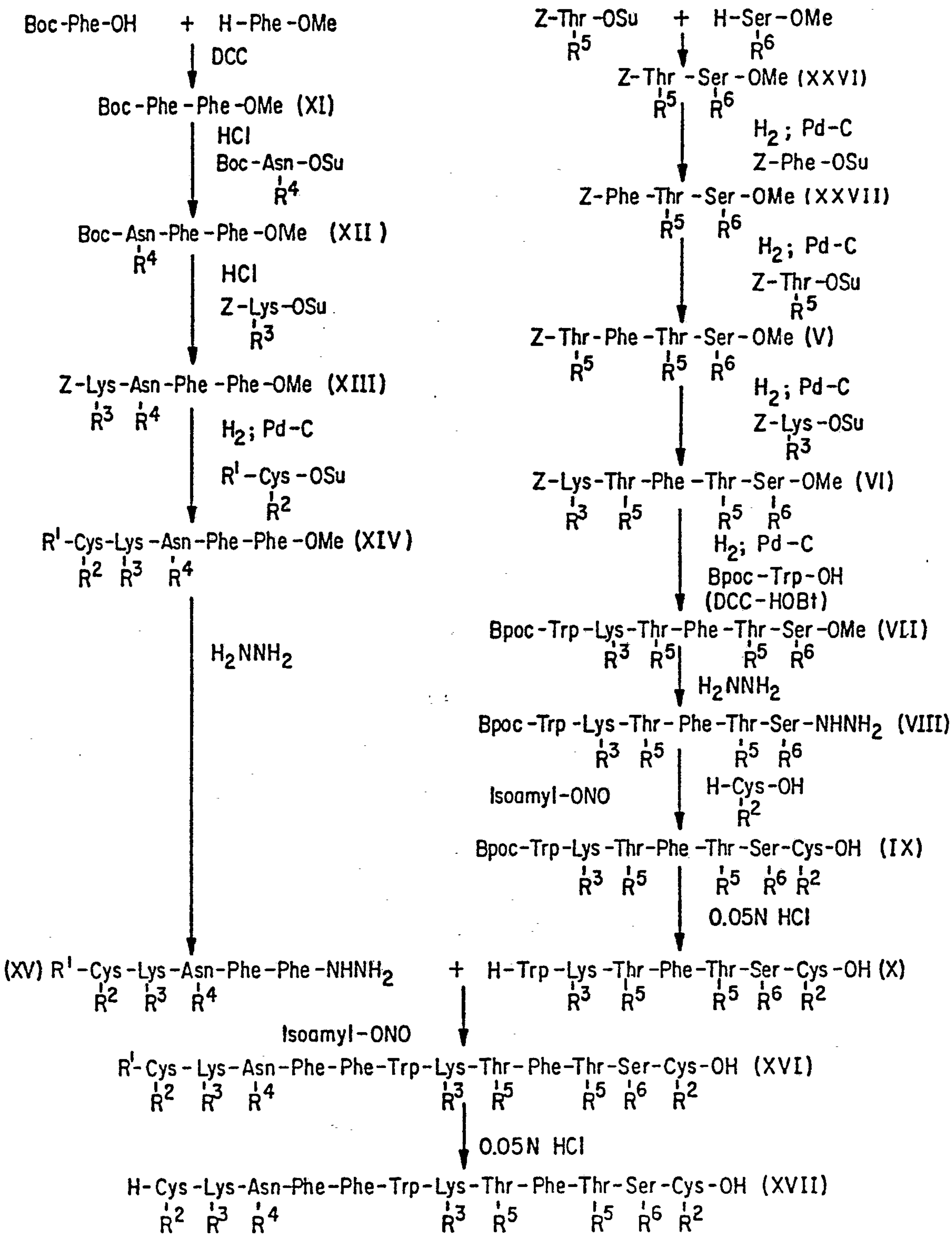
DCC
(XI)
HCl
Boc-Asn-OSu
(XII)
HCl
Z-Lys-OSu
(XIII)
$H_2$; Pd-C
(XIV)
$H_2NNH_2$
(XV)
(XXVI)
$H_2$; Pd-C
Z-Phe-OSu
(XXVII)
$H_2$; Pd-C
Z-Thr-OSu
(V)
$H_2$; Pd-C
Z-Lys-OSu
(VI)
$H_2$; Pd-C
Bpoc-Trp-OH
(DCC-HOBt)
(VII)
$H_2NNH_2$
(VIII)
Isoamyl-ONO
H-Cys-OH
(IX)
0.05N HCl
(X)
Isoamyl-ONO
(XVI)
0.05N HCl
(XVII)

FIGUR 2

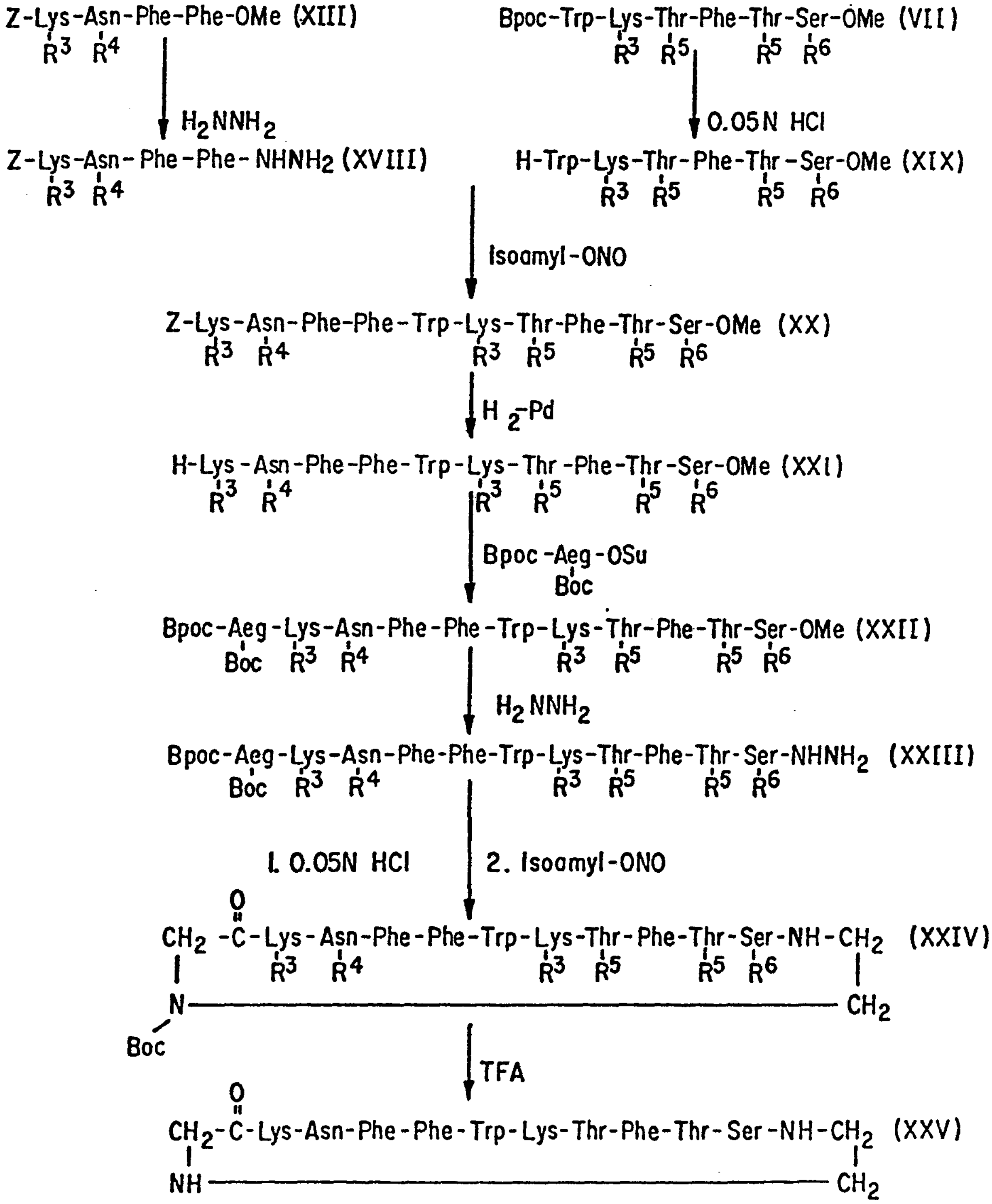


(XIII) (VII) (XVIII) (XIX) (XX) (XXI) (XXII) (XXIII) (XXIV) (XXV)

**Patentansprüche**

1. Polypeptide der Formel

X-Lys-Asn-Phe-Phe-A-Lys-Thr-Phe-Thr-Ser-Y I

worin

A L- oder D-Trp,

X H-$(Aeg)_m$-Cys- oder H-$(Aeg)_m$-Ala-Gly-Cys-,

Y -Cys-$(Aeg)_n$-OH oder

X und Y zusammen eine 2-Aminoäthylglycylgruppe in Ringposition und

m und n 0, 1, 2, 3 oder 4 bedeuten, unter der Voraussetzung, dass m + n mindestens 1 ist, deren cyclische Disulfidderivate, Protamin-Zink- und Protamin-Aluminium-Komplexe und physiologisch verträgliche Säureadditionssalze.

2. H-Aeg-Cys-Lys-Asn-Phe-Phe-Trp-Lys-Thr-Phe-Thr-Ser-Cys-OH.

3. H-Aeg-Cys-Lys-Asn-Phe-Phe-Trp-Lys-Thr-Phe-Thr-Ser-Cys-OH.

4. H-Aeg-Aeg-Cys-Lys-Asn-Phe-Phe-Trp-Lys-Thr-Phe-Thr-Ser-Cys-OH.

5. H-Aeg-Ala-Gly-Cys-Lys-Asn-Phe-Phe-Trp-Lys-Thr-Phe-Thr-Ser-Cys-OH.

6. Aeg-Lys-Asn-Phe-Phe-Trp-Lys-Thr-Phe-Thr-Ser

7. H-Aeg-Cys-Lys-Asn-Phe-Phe-D-Trp-Lys-Thr-Phe-Thr-Ser-Cys-OH.

8. H-Aeg-Cys-Lys-Asn-Phe-Phe-D-Trp-Lys-Thr-Phe-Thr-Ser-Cys-OH.

9. H-Aeg-Aeg-Cys-Lys-Asn-Phe-Phe-D-Trp-Lys-Thr-Phe-Thr-Ser-Cys-OH.

10. H-Aeg-Aeg-Cys-Lys-Asn-Phe-Phe-D-Trp-Lys-Thr-Phe-Thr-Ser-Cys-OH.

11. H-Cys-Lys-Asn-Phe-Phe-Trp-Lys-Thr-Phe-Thr-Ser-Cys-Aeg-OH.

12. H-Cys-Lys-Asn-Phe-Phe-D-Trp-Lys-Thr-Phe-Thr-Ser-Cys-Aeg-OH.

13. H-Aeg-Cys-Lys-Asn-Phe-Phe-Trp-Lys-Thr-Phe-Thr-Ser-Cys-Aeg-OH.

14. H-Aeg-Cys-Lys-Asn-Phe-Phe-D-Trp-Lys-Thr-Phe-Thr-Ser-Cys-Aeg-OH.

15. Verfahren zur Herstellung von Polypeptiden der Formel

X-Lys-Asn-Phe-Phe-A-Lys-Thr-Phe-Thr-Ser-Y I

worin

A L- oder D-Trp,

X H-$(Aeg)_m$-Cys- oder H-$(Aeg)_m$-Ala-Gly-Cys-,

Y -Cys-$(Aeg)_n$-OH oder

X und Y zusammen eine 2-Aminoäthylglycylgruppe in Ringposition und

m und n 0, 1, 2, 3 oder 4 bedeuten, unter der Voraussetzung, dass m + n mindestens 1 ist, und von deren cyclischen Disulfidderivaten, Protamin-Zink- und Protamin-Aluminium-Komplexen sowie physiologisch verträglichen Säureadditionssalzen, dadurch gekennzeichnet, dass man in an sich bekannter Weise nach der Flüssigphasen- oder nach der Festphasen-Technik arbeitet und gewünschtenfalls eine erhaltene Verbindung der Formel I mild oxidiert zum entsprechenden cyclischen Disulfid und/oder in einen Protamin-Zink- oder Protamin-Aluminium-Komplex oder ein physiologisch verträgliches Säureadditionssalz überführt.

16. Verfahren gemäss Anspruch 15, dadurch gekennzeichnet, dass man in der letzten Stufe einer konventionellen Flüssigphasen-Synthese die Schutzgruppen aus einem geschützten Polypeptid der Formel

X'-Lys($R^3$)-Asn($R^4$)-Phe-Phe-A-Lys($R^3$)-Thr($R^5$)-Phe-Thr($R^5$)-Ser($R^6$)-Y' II

worin

A L- oder D-Trp

X' $R^1$-$(Aeg)_m$-Cys($R^2$)- oder
$R^1$-$(Aeg)_m$-Ala-Gly-Cys($R^2$)-;

Y' -Cys($R^2$)-$(Aeg)_m$-OH oder

X' und Y' zusammen eine gegebenenfalls geschützte 2-Aminoäthylglycyl-gruppe in Ringposition;

$R^1$ Wasserstoff oder eine konventionelle $\alpha$-Aminoschutzgruppe,

$R^2$ eine konventionelle Schutzgruppe für die Sulfhydrylgruppe des Cysteins;

$R^3$ eine konventionelle Schutzgruppe für die $\varepsilon$-Aminogruppe des Lysins;

$R^4$ Wasserstoff oder eine konventionelle Schutzgruppe für die Carboxamidgruppe des Asparagins;
$R^5$ Wasserstoff oder eine konventionelle Schutzgruppe für die Hydroxylgruppe des Threonins;
$R^6$ Wasserstoff oder eine konventionelle Schutzgruppe für die Hydroxylgruppe des Serins und
m und n 0, 1, 2, 3 oder 4 darstellen, unter der Voraussetzung, dass m + n mindestens 1 ist, abspaltet.

17. Verfahren zur Herstellung von pharmazeutischen Präparaten bei dem die Wirksubstanz mit einem zur therapeutischen Verabreichung geeigneten, inerten, festen oder flüssigen Träger vermischt und das Gemisch in eine geeignete galenische Form gebracht wird, dadurch gekennzeichnet, dass als Wirksubstanz eine Verbindung gemäss Anspruch 1 verwendet wird.

18. Pharmazeutische Präparate auf der Basis einer Verbindung gemäss Anspruch 1.

**Revendications**

1. Un polypeptide de formule

X-Lys-Asn-Phe-Phe-A-Lys-Thr-Phe-Thr-Ser-Y    I

où

A = L- ou D-Trp,
X = H-$(Aeg)_m$-Cys- ou H-$(Aeg)_m$-Ala-Gly-Cys-,
Y = -Cys-$(Aeg)_n$-OH ou
X et Y pris ensemble représentent un groupe 2-aminoéthyl-glycyle en position de cycle, et
m et n = 0, 1, 2, 3 ou 4, à condition que
m + n = au moins 1,

les dérivés disulfide cycliques, les complexes protamine-zinc et protamine-aluminium et leurs sels d'addition d'acides physiologiquement acceptables.

2. H-Aeg-Cys-Lys-Asn-Phe-Phe-Trp-Lys-Thr-Phe-Thr-Ser-Cys-OH.
3. H-Aeg-Cys-Lys-Asn-Phe-Phe-Trp-Lys-Thr-Phe-Thr-Ser-Cys-OH.
4. H-Aeg-Aeg-Cys-Lys-Asn-Phe-Phe-Trp-Lys-Thr-Phe-Thr-Ser-Cys-OH.
5. H-Aeg-Ala-Gly-Cys-Lys-Asn-Phe-Phe-Trp-Lys-Thr-Phe-Thr-Ser-Cys-OH.
6. Aeg-Lys-Asn-Phe-Phe-Trp-Lys-Thr-Phe-Thr-Ser.
7. H-Aeg-Cys-Lys-Asn-Phe-Phe-D-Trp-Lys-Thr-Phe-Thr-Ser-Cys-OH.
8. H-Aeg-Cys-Lys-Asn-Phe-Phe-D-Trp-Lys-Thr-Phe-Thr-Ser-Cys-OH.
9. H-Aeg-Aeg-Cys-Lys-Asn-Phe-Phe-D-Trp-Lys-Thr-Phe-Thr-Ser-Cys-OH.
10. H-Aeg-Aeg-Cys-Lys-Asn-Phe-Phe-D-Trp-Lys-Thr-Phe-Thr-Ser-Cys-OH.
11. H-Cys-Lys-Asn-Phe-Phe-Trp-Lys-Thr-Phe-Thr-Ser-Cys-Aeg-OH.
12. H-Cys-Lys-Asn-Phe-Phe-D-Trp-Lys-Thr-Phe-Thr-Ser-Cys-Aeg-OH.
13. H-Aeg-Cys-Lys-Asn-Phe-Phe-Trp-Lys-Thr-Phe-Thr-Ser-Cys-Aeg-OH.
14. H-Aeg-Cys-Lys-Asn-Phe-Phe-D-Trp-Lys-Thr-Phe-Thr-Ser-Cys-Aeg-OH.
15. Un procédé pour la préparation de polypeptides de formule

X-Lys-Asn-Phe-Phe-A-Lys-Thr-Phe-Thr-Ser-Y    I

où

A = L- ou D-Trp,
X = H-$(Aeg)_m$-Cys- ou H-$(Aeg)_m$-Ala-Gly-Cys-,
Y = -Cys-$(Aeg)_n$-OH ou
X et Y pris ensemble représentent un groupe 2-aminoéthyl-glycyle en position de cycle, et
m et n = 0, 1, 2, 3 ou 4, à condition que
m + n = au moins 1,

et de leurs dérivés disulfide cycliques, complexes protamine-zinc et protamine-aluminium et sels d'addition d'acides physiologiquement acceptables, caractérisé en ce qu'on utilise des méthodes conventionnelles de synthèse en phase liquide ou solide et, le cas échéant, on soumet le composé de formule I à oxydation douce afin de réaliser la formation du disulfide cyclique correspondant, et/ou convertit en un complexe protamine-zinc ou protamine-aluminium ou un sel d'addition d'acide physiologiquement acceptable.

16. Un procédé selon la revendication 15, caractérisé en ce que la dernière étape d'une synthèse

de phase liquide conventionnelle comprend la scission des groupes protecteurs d'un polypeptide protégé de formula

$$X'\text{-Lys}(R^3)\text{-Asn}(R^4)\text{-Phe-Phe-A-Lys}(R^3)\text{-Thr}(R^5)\text{-Phe-Thr}(R^5)\text{-Ser}(R^6)\text{-}Y' \qquad II$$

où

A = L- ou D-Trp,
X' = $R^1$-$(Aeg)_m$-Cys($R^2$)- ou
$R^1$-$(Aeg)_m$-Ala-Gly-Cys($R^2$)-;
Y' = -Cys($R^2$)-$(Aeg)_m$-OH ou
X' et Y' pris ensemble représentent un groupe 2-aminoéthyl-glycyle ou 2-(amino protégé)-éthyl-glycyle en position de cycle;
$R^1$ = H ou un groupe protecteur conventionnel du groupe $\alpha$-amino;
$R^2$ = un groupe protecteur conventionnel pour le groupe sulfhydryle de la cystéine;
$R^3$ = un groupe protecteur conventionnel pour le groupe $\varepsilon$-amino de la lysine;
$R^4$ = H ou un groupe protecteur conventionnel pour le groupe carboxamide de l'asparagine;
$R^5$ = H ou un groupe protecteur conventionnel pour le groupe hydroxyle de la thréonine;
$R^6$ = H ou un groupe protecteur conventionnel pour le groupe hydroxyle de la sérine, et
m et n = 0, 1, 2, 3 ou 4, à condition que
m + n = au moins 1.

17. Un procédé pour la préparation de compositions pharmaceutiques consistant à mélanger la substance active avec un support solide ou liquide inerte, thérapeutiquement compatible et transformer le mélange en une forme galénique appropriée, caractérisé en ce que la substance active est un composé selon la revendication 1.

18. Compositions pharmaceutiques sur la base d'un composé selon la revendication 1.

**Claims**

1. A polypeptide of the formula

$$\text{X-Lys-Asn-Phe-Phe-A-Lys-Thr-Phe-Thr-Ser-Y} \qquad I$$

wherein

A is L- or D-Trp,
X is H-$(Aeg)_m$-Cys- or H-$(Aeg)_m$-Ala-Gly-Cys-,
Y is -Cys-$(Aeg)_n$-OH or
X and Y taken together are a 2-aminoethylglycyl group in the ring position and
m and n are 0, 1, 2, 3 or 4, provided that
m + n are at least 1,

the cyclic disulfide derivatives, protamine zinc and protamine aluminum complexes and physiologically acceptable acid addition salts thereof.

2. H-Aeg-Cys-Lys-Asn-Phe-Phe-Trp-Lys-Thr-Phe-Thr-Ser-Cys-OH.
3. H-Aeg-Cys-Lys-Asn-Phe-Phe-Trp-Lys-Thr-Phe-Thr-Ser-Cys-OH (Cys–Cys bridged).
4. H-Aeg-Aeg-Cys-Lys-Asn-Phe-Phe-Trp-Lys-Thr-Phe-Thr-Ser-Cys-OH.
5. H-Aeg-Ala-Gly-Cys-Lys-Asn-Phe-Phe-Trp-Lys-Thr-Phe-Thr-Ser-Cys-OH.
6. Aeg-Lys-Asn-Phe-Phe-Trp-Lys-Thr-Phe-Thr-Ser (cyclic).
7. H-Aeg-Cys-Lys-Asn-Phe-Phe-D-Trp-Lys-Thr-Phe-Thr-Ser-Cys-OH.
8. H-Aeg-Cys-Lys-Asn-Phe-Phe-D-Trp-Lys-Thr-Phe-Thr-Ser-Cys-OH (Cys–Cys bridged).
9. H-Aeg-Aeg-Cys-Lys-Asn-Phe-Phe-D-Trp-Lys-Thr-Phe-Thr-Ser-Cys-OH.
10. H-Aeg-Aeg-Cys-Lys-Asn-Phe-Phe-D-Trp-Lys-Thr-Phe-Thr-Ser-Cys-OH (Cys–Cys bridged).
11. H-Cys-Lys-Asn-Phe-Phe-Trp-Lys-Thr-Phe-Thr-Ser-Cys-Aeg-OH (Cys–Cys bridged).
12. H-Cys-Lys-Asn-Phe-Phe-D-Trp-Lys-Thr-Phe-Thr-Ser-Cys-Aeg-OH.
13. H-Aeg-Cys-Lys-Asn-Phe-Phe-Trp-Lys-Thr-Phe-Thr-Ser-Cys-OH.
14. H-Aeg-Cys-Lys-Asn-Phe-Phe-D-Trp-Lys-Thr-Phe-Thr-Ser-Cys-Aeg-OH.
15. A process for the preparation of polypeptides of the formula

$$\text{X-Lys-Asn-Phe-Phe-A-Lys-Thr-Phe-Thr-Ser-Y} \qquad I$$

wherein

A is L- or D-Trp,

X is H-$(Aeg)_m$-Cys- or H-$(Aeg)_m$-Ala-Gly-Cys-,

Y is -Cys-$(Aeg)_n$-OH or

X and Y taken together are a 2-aminoethylglycyl group in the ring position and

m and n are 0, 1, 2, 3 or 4, provided that

m + n are at least 1,

and of the cyclic disulfide derivatives, protamine zinc and protamine aluminum complexes and physiologically acceptable acid addition salts thereof, which process comprises using conventional liquid phase synthesis methods or solid phase synthesis methods and, if desired, oxidizing mildly the compound of formula I obtained to achieve the formation of the corresponding cyclic disulfide and/or forming a zinc or an aluminum protamine complex or a physiologically acceptable acid addition salt thereof.

16. A process as claimed in claim 15, characterized in that the last step of a conventional liquid phase synthesis comprises cleaving off the protecting groups of a protected polypeptide of the formula

$$X'\text{-Lys}(R^3)\text{-Asn}(R^4)\text{-Phe-Phe-A-Lys}(R^3)\text{-Thr}(R^5)\text{-Phe-Thr}(R^5)\text{-Ser}(R^6)\text{-}Y' \qquad II$$

wherein

A is L- or D-Trp

X′ is $R^1$-$(Aeg)_m$-Cys($R^2$)- or

$R^1$-$(Aeg)_m$-Ala-Gly-Cys($R^2$)-;

Y′ is -Cys($R^2$)-$(Aeg)_m$-OH or

X′ and Y′ taken together are a 2-aminoethylglycyl or 2-(protected amino)-ethylglycyl group in the ring position;

$R^1$ is hydrogen or a conventional $\alpha$-amino protecting group;

$R^2$ is a conventional protecting group for the sulfhydryl group of cystein;

$R^3$ is a conventional protecting group for the $\varepsilon$-amino group of lysine;

$R^4$ is hydrogen or a conventional protecting group for the carboxamide group of asparagine;

$R^5$ is hydrogen or a conventional protecting group for the hydroxyl group of threonine;

$R^6$ is hydrogen or a conventional protecting group for the hydroxyl group of serine and

m and n are 0, 1, 2, 3 or 4, provided that

m + n are at least 1.

17. Process for the manufacture of pharmaceutical preparations by mixing the active substance with an inert, therapeutically compatible solid or liquid carrier and bringing the mixture into a suitable galenic form, characterized in that the active substance is a compound in accordance with claim 1.

18. Pharmaceutical preparations on the basis of a compound in accordance with claim 1.